# EUROPEAN PATENT SPECIFICATION

(11) **EP 4 724 116 B1**
(45) Date of publication and mention of the grant of the patent: **12.08.2026**
(21) Application number: 23825464.3
(22) Date of filing: 30.11.2023
(51) Int. Cl.: A61L 27/26

(54) **SCAFFOLD FOR CORNEAL REGENERATION AND METHOD FOR PRODUCING THE SAME**
GERÜST ZUR HORNHAUTREGENERATION UND VERFAHREN ZUR HERSTELLUNG DAVON
ÉCHAFAUDAGE DESTINÉ À LA RÉGÉNÉRATION CORNÉENNE ET SA MÉTHODE DE PRODUCTION

(43) Date of publication of application: 15.04.2026
(73) Proprietor: MEDEZE TREASURY PTE. LTD, Singapore 117406 (SG)
(72) Inventor: KHEMARANGSAN, Veerapol, Singapore 117406 (SG)
(74) Representative: Curell Suñol S.L.P.
(86) International application number: PCT/IB2023/062042
(87) International publication number: WO 2025/114753

(56) References cited:
- CN-A- 111 560 709
- JEONG IN KIM: "Fabrication of transparent hemispherical 3D nanofibrous scaffolds with radially aligned patterns via a novel electrospinning method", SCIENTIFIC REPORTS, vol. 8, no. 1, 21 February 2018 (2018-02-21), US, XP093167079, ISSN: 2045-2322, Retrieved from the Internet <URL:https://www.nature.com/articles/s41598-018-21618-0.pdf> DOI: 10.1038/s41598-018-21618-0
- ANKUSH DEWLE ET AL: "Multifarious Fabrication Approaches of Producing Aligned Collagen Scaffolds for Tissue Engineering Applications", HHS AUTHOR MANUSCRIPTS, vol. 6, no. 2, 14 January 2020 (2020-01-14), US, pages 779 - 797, XP055727287, DOI: 10.1021/acsbiomaterials.9b01225
- KONG BIN ET AL: "Electrospun Scaffolds for Corneal Tissue Engineering: A Review", MATERIALS, vol. 9, no. 8, 27 July 2016 (2016-07-27), pages 614, XP055853508, Retrieved from the Internet <URL:https://www.ncbi.nlm.nih.gov/pmc/articles/PMC5509008/pdf/materials-09-00614.pdf> DOI: 10.3390/ma9080614

## Description

### TECHNICAL FIELD

The invention presented herein is generally directed toward a scaffold for corneal regeneration. More particularly, a scaffold for corneal regeneration and a method to produce thereof.

### BACKGROUND

Approximately 4.9 million individuals worldwide are affected by corneal blindness. However, the primary treatment method, which involves allogenic corneal transplantations, faces significant challenges such as immunological rejection and a severe shortage of donor corneas. Therefore, it is crucial to explore alternative treatment approaches and corneal substitutes to overcome these issues. Tissue engineering has emerged as a promising solution for this purpose.

An ideal tissue-engineered construct for corneal treatment should closely replicate the native cornea's stiffness and its ability to transmit and focus light. It must also be biocompatible, biodegradable, and possess a robust structure that mimics the cornea's natural environment, providing temporary support during tissue regeneration. The cornea primarily consists of the stroma, a highly organized network of collagen-type I nanofibrils. These nanofibrils are uniformly spaced and aligned in parallel, forming approximately 200 lamellae sheets, each oriented orthogonally to its neighbors. This unique structure minimizes light scattering and maximizes light transmission, contributing to high transparency. Additionally, the variation in lamellae thickness and width, along with their fusion, enhances stromal integrity. Consequently, it is essential for engineered corneal tissue to replicate these properties.

Nanostructured scaffolds made from natural or synthetic materials are considered ideal for corneal replacements, as they closely mimic the native corneal fibril structure and biological function. Over the past few years, electrospinning has emerged as a versatile technique for creating nanoengineered corneal constructs. By adjusting electrospinning parameters, it's possible to fabricate fibers of varying material compositions, diameters, and arrangements, resulting in scaffolds that exhibit mechanical strength, transparency, and aligned fibers resembling the corneal stroma. Additionally, nanofibrous scaffolds feature high porosity and surface area, similar to the collagen fibers in the corneal extracellular matrix.

Regarding scaffold materials, natural polymers are commonly preferred for their biocompatibility and biodegradability. Collagen type I, the main component of the natural cornea, is frequently used. However, collagen has drawbacks, including instability and rapid degradation, as well as lower mechanical properties due to purification processes. Polycaprolactone (PCL), a synthetic polymer, offers advantages such as stability, consistent quality, and greater mechanical strength compared to collagen. Therefore, an optimal approach involves creating a hybrid scaffold that combines the biofunctional benefits of collagen with the stability of PCL. This combination ensures the strength, durability, and bioactivity required for tissue-engineered corneal scaffolds. Kim et al. Scientific Reports, vol. 8, no. 1, 21 February 2018 discloses corneal scaffolds with aligned nanofibers of PCL/collagen.

This specification recognizes that there is a need for a hybrid electrospun scaffold for corneal tissue engineering consisting of PCL and type I collagen, evaluating it in terms of light transmittance, stability, and mechanical properties.

Thus, in view of the above, there is a long-felt need in the industry to address the aforementioned deficiencies.

It is with respect to these and other considerations that the disclosure made herein is presented.

### SUMMARY

A scaffold for corneal regeneration and a method to produce thereof are provided, as shown in and/or described in connection with at least one of the figures.

One aspect of the present disclosure relates to a method for producing a scaffold for corneal generation, the method comprising a step (a) of deriving a layer of aligned fibers from a solution comprising a biocompatible polycaprolactone (PCL) and a collagen, wherein the PCL and collagen have a mass ratio in a range of 77:23 to 42:58. The method includes a step (b) of using a first crosslinker to crosslink the aligned fibers in the layer obtained from the step (a). The method includes a step (c) of applying a plasma treatment to the aligned fibers in the layer obtained from the step (b). The method includes a step (d) using a second crosslinker to crosslink the aligned fibers in the layer obtained from the step (c). The method further includes a step (e) of stacking a plurality of the layers of the aligned fibers obtained from the step (d) to produce the scaffold comprising a plurality of the layers of the aligned fibers.

In an aspect, the layer of the aligned fibers has a thickness that is preferably from 15 to 20 µm.

In an aspect, the scaffold preferably comprises four layers of the aligned fibers.

In an aspect, the first crosslinker is selected from the group consisting of 1-Ethyl-3-(3-dimethylaminopropyl) carbodiimide and N-Hydroxysuccinimide (EDC/NHS), Glutaraldehyde, Riboflavin, Genipin, Paraformaldehyde, and any combination thereof.

In an aspect, the first crosslinker is preferably 1-Ethyl-3-(3-dimethylaminopropyl) carbodiimide and N-Hydroxysuccinimide (EDC/NHS).

In an aspect, the second crosslinker is selected from the group consisting of 1-Ethyl-3-(3-dimethylaminopropyl) carbodiimide and N-Hydroxysuccinimide (EDC/NHS), Glutaraldehyde, Riboflavin, Genipin, Paraformaldehyde, and any combination thereof.

In an aspect, the second crosslinker is preferably 1-Ethyl-3-(3-dimethylaminopropyl) carbodiimide and N-Hydroxysuccinimide (EDC/NHS).

In an aspect, the plasma treatment is carried out with an oxygen gas stream at a flow rate of 50 standard cubic centimeters per minute (sccm) and for 20 to 119 minutes.

In an aspect, the scaffold has various properties that include but are not limited to a visible light transmittance in the range of 50 to 95%; a tensile strength less than or equal to 19.1 MPa; and an elasticity modulus in the range of 1 MPa to 34 MPa. Another aspect of the invention relates to a scaffold for corneal regeneration comprising at least one layer of aligned fibers, wherein said at least one layer of aligned fibers is obtainable by a method according to the fist aspect of the invention and its specific embodiments according to the other aspects above.

Other embodiments and advantages will become readily apparent to those skilled in the art upon viewing the drawings and reading the detailed description hereafter, all without departing from the scope of the claims. The drawings and detailed descriptions presented are to be regarded as illustrative in nature and not in any way as restrictive.

Other features of the example embodiments will be apparent from the drawings and from the detailed description that follows.

### BRIEF DESCRIPTION OF THE DRAWINGS

The detailed description is set forth with reference to the accompanying drawings. The use of the same reference numerals may indicate similar or identical items. Various embodiments may utilize elements and/or components other than those illustrated in the drawings, and some elements and/or components may not be present in various embodiments. Elements and/or components in the figures are not necessarily drawn to scale. Throughout this disclosure, depending on the context, singular and plural terminology may be used interchangeably.
FIG. 1 illustrates a schematic diagram of an electrospinning process of PCL-COL scaffolds on a grid or drum collector (interchangeable), in accordance with at least one embodiment.
FIG. 2A illustrates a schematic diagram showing the crosslinking process, in accordance with at least one embodiment.
FIG. 2B illustrates a schematic diagram showing the plasma treatment process, in accordance with at least one embodiment.
FIG. 3 illustrates (A) FTIR spectrum of electrospun pure PCL scaffold, with unique peaks at 2945, 1720 and 1165cm⁻¹; (B) FTIR spectrum of electrospun pure collagen scaffold, with unique peaks at 3300, 1650, and 1550 cm⁻¹; (C) FTIR spectrum of PCL-collagen scaffold, with unique peaks at 2945, 1720 and 1165 cm⁻¹ matching PCL, and 3300, 1650 and 1550 cm⁻¹ matching collagen.
FIGS. 4A-4B illustrate various perspective views of the scaffold with a mass ratio of PCL and collagen 100:0 (4A) and 80:20 (4B), which are white, opaque, and relatively hydrophobic, shriveling up in PBS.
FIGS. 4C-4D illustrate various perspective views of 2500x magnification SEM images of scaffold with a mass ratio of PCL and collagen 0:100 (4C) and 40:60 (4D), which the fibers are not of good quality, with multiple droplets.
FIG. 4E illustrates a perspective view of a poor-quality scaffold produced when the PCL: collagen ratio is 40:60, which is thin, fragile, and full of droplets.
FIG. 5 illustrates perspective views of SEM images of PCL-collagen scaffolds with various mass ratios.
FIG. 6A illustrates a perspective view of the diameter range histogram of electrospun PCL-collagen scaffolds spun using the grid collector.
FIG. 6B illustrates a perspective view of the SEM image of PCL-collagen scaffolds spun using the grid collector (mass ratio 77:23), in accordance with at least one embodiment.
FIG. 6C illustrates a perspective view of the diameter range histogram of electrospun PCL-collagen scaffolds spun using the drum collector, in accordance with at least one embodiment.
FIG. 6D illustrates a perspective view of the SEM image of PCL-collagen scaffolds spun using the drum collector (mass ratio 77:23), in accordance with at least one embodiment.
FIG. 7 illustrates a graphical representation of a stress-strain curve of the scaffolds of PCL-collagen at a mass ratio of 49:51 which are crosslinked twice in EDC/NHS for 5 hours both before and after plasma treatment, in accordance with at least one embodiment.
FIG. 8 illustrates a graphical representation of stress-strain graphs of PCL-collagen scaffolds (mass ratio of 68:32) electrospun with the drum collector and crosslinked by 0.2% glutaraldehyde solution.
FIG. 9 illustrates a perspective view of a comparison of peeling the scaffolds after 18 hours vs. 5 hours of EDC/NHS crosslinking at 25 °C, in accordance with at least one embodiment.
FIG. 10 illustrates a graphical representation of a graph of transmittance over wavelength for 300-layer scaffolds spun with the grid collector.
FIG. 11 illustrates a graphical representation of the effect of plasma treatment on the light transmittance of PCL-collagen scaffolds electrospun with the drum collector (mass ratio 77:23).
FIG. 12 illustrates a perspective view of scaffolds spun on a grid collector and collected on a glass cover slip, before and after plasma treatment with conditions 20 W, 80 kHz, and 40 minutes.
FIG. 13 illustrates a graphical representation of the effect of plasma duration on the light transmittance of non-crosslinked PCL-collagen scaffolds electrospun with the drum collector (mass ratio 68:32).
FIG. 14 illustrates a graphical representation of the effect of plasma duration on the light transmittance of EDC/NHS cross-linked PCL-collagen scaffolds electrospun with the drum collector (mass ratio 49:51).
FIG. 15 illustrates a graphical representation of transparency (top) and stress-strain curves of unstack scaffolds after the first crosslinking or 1^{st} crosslinking (bottom left) vs. the second crosslinking or 2^{nd} crosslinking (bottom right), in accordance with at least one embodiment.
FIG. 16 illustrates a graphical representation of the effect of plasma treatment on the light transmittance of PCL-collagen scaffolds electrospun with the drum collector (mass ratio 68:32) after plasma treatment at 70 kHz, 20 W, 30 min.
FIG. 17 illustrates a perspective view of scaffolds that can be cut into all kinds of shapes, like round and rectangular ones.
FIG. 18 illustrates images from animal study trials on preserved rabbits.
FIG. 19 illustrates a perspective view of the processes of stacking 4 layers of electrospun scaffolds to obtain the prototype for animal study (top), and the stacked 4-layer scaffolds can be sutured (bottom, left) and trimmed with scissors (bottom, right), being suitable for implantation in the injured eyes of animals.
FIG. 20 is a flowchart of a method for producing a scaffold for corneal generation, in accordance with one or more example embodiments.
FIG. 21 illustrates a graphical representation of stress-strain graphs of a PCL-collagen scaffold sample (mass ratio of 49:51) electrospun with the drum collector and crosslinked by EDC/NHS solution, obtained with in-house test methods with Young's Modulus or elasticity modulus of approximately 7.30 MPa.
FIG. 22 illustrates a graphical representation of stress-strain graphs of a PCL-collagen scaffold sample (mass ratio of 49:51) electrospun with the drum collector and the first crosslinked by EDC/NHS solution, obtained from Instron testing trial 1.
FIG. 23 illustrates a graphical representation of stress-strain graphs of a PCL-collagen scaffold sample (mass ratio of 49:51) electrospun with the drum collector and the second crosslinked by EDC/NHS solution, obtained from Instron testing trial 2.
FIG. 24 illustrates a graphical representation of instron trial 1 average stress-strain graphs of PCL-collagen scaffolds (mass ratio of 49:51) electrospun with the drum collector and crosslinked by EDC/NHS solution.
FIG. 25 illustrates a graphical representation of instron trial 2 average Stress-strain graphs of PCL-collagen scaffolds (mass ratio of 49:51) electrospun with the drum collector and crosslinked by EDC/NHS solution.
FIG. 26 illustrates images of pre-implantation and post-implantation of the scaffold grafts into rabbit eyes, in accordance with at least one embodiment.
FIG. 27A-D illustrates graphical representations of complete blood count levels for: (A) white blood cells (WBC); (B) neutrophils (PMN); (C) lymphocytes (LYM); and (D) monocytes (Mono) at pre-implantation (n=6) and surgical post-implantation (28 days after implantation, n=6), in accordance with at least one embodiment.
FIG. 28A-G illustrates graphical representations of blood chemistry levels for: (A) creatinine (CRE); (B) albumin; (C) total protein (TP); (D) glucose (GLU); (E) ALT (SGPT); (F) alkaline phosphatase (ALP); and (G) blood urea nitrogen (BUN) at pre-implantation (n=6) and surgical post-implantation (28 days after implantation, n=6), in accordance with at least one embodiment.

### DETAILED DESCRIPTION

The disclosure will be described more fully hereinafter with reference to the accompanying drawings, in which example embodiments of the disclosure are shown, and not intended to be limiting.

Aspects of the present disclosure relate to a scaffold for corneal regeneration that includes at least one layer of aligned fibers. The aligned fibers include a biocompatible polycaprolactone (PCL) and a collagen. The PCL and the collagen have a mass ratio in the range of 77:23 to 42:58. In an embodiment, each layer of the aligned fibers has a thickness that is preferably from 15 to 20 µm. The scaffold preferably includes four layers of the aligned fibers. The collagen is type I collagen derived from a human collagen, a bovine collagen, a porcine collagen, or an aquatic animal collagen. The type I collagen is preferably an Atelocollagen derived from the bovine collagen.

### Materials And Methods:

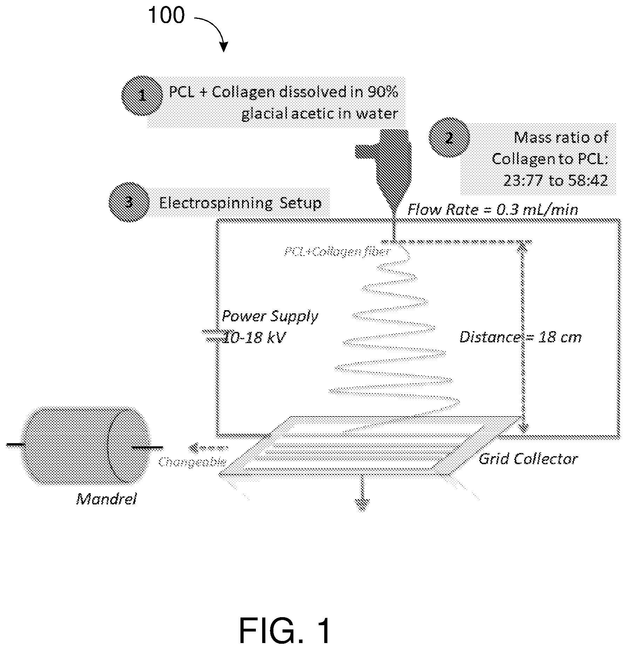

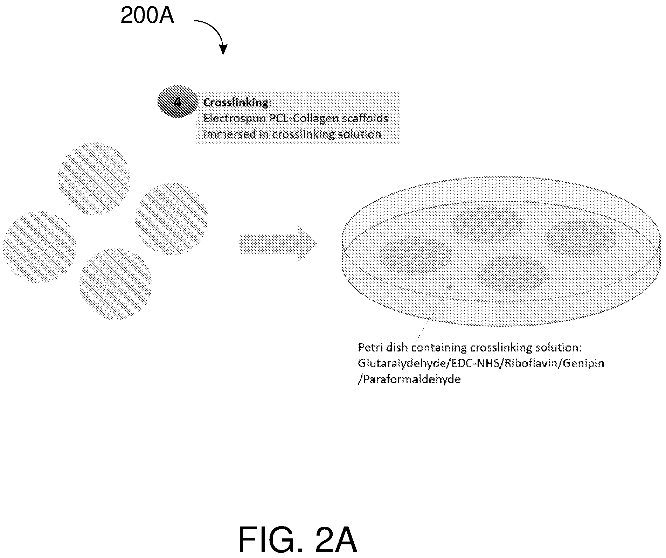

Electrospinning of scaffolds with aligned fibers: The solvent used for all dissolving processes is 90% glacial acetic acid, prepared by diluting one part of distilled water into nine parts of glacial acetic acid (Merk, Thailand). A solution of PCL was prepared by dissolving PCL pellets (molecular weight: 80,000 g/mol, Sigma-Aldrich, Singapore) at a concentration of 23% (w/v). Separately, a solution of collagen was prepared by dissolving type I collagen from bovine tendon (Koken Co. Ltd., Japan) at a concentration of 16% (w/v). The two solutions are subsequently mixed in the volume ratio of PCL: Collagen 4:6 (mass ratio 46:48) for electrospinning with the rotating drum, or 7:3 (mass ratio 77:23) for grid collector, to form a PCL-collagen blend solution (PCL-COL). Ideally, the range of mass ratios of PCL and collagen must be between 77:23 and 42:58. (Table 1A and 1B) These ranges of mixture tend to generate good quality fibers and scaffolds (FIG. 1). Mixtures that are out of these ranges would result in undesirable scaffolds (FIG. 2A-2B).

Electrospinning was conducted with the NS1 Double NanoSpinner Electrospinning Device (Inovenso, Turkey). The PCL-COL solution was aspirated into a 5 mL syringe (BD, Singapore) fixed to a stainless-steel needle (Nominal I.D.: 0.514mm, Inovenso, Turkey) connected to a high-voltage power supply. The flow rate was set to 0.3mL/h and the applied voltage has a range of 10 to 18 kV. This condition allows the solution to be transformed into fibrous solid scaffolds. There are two methods by which scaffolds with aligned fibers can be fabricated: rotating drum and grid collector (FIG. 1).

FIG. 1 illustrates a schematic diagram 100 of an electrospinning process of PCL-COL scaffolds on a drum or grid collector (interchangeable), in accordance with at least one embodiment. For the former, the electrospun fibers were collected onto a piece of aluminum foil on a drum collector rotating at 1200 RPM for 10 hours, situated about 18 cm from the tip of the needle. Scaffolds can also be produced after 8h, 9h, and 12h of electrospinning, between 1200 and 2000 RPM (Table 1A). Aligned scaffolds were then peeled off from the foil and cut into circular sheets of 25 mm (diameter) using a circle punch cutter.

**Table 1A: List of mass ratios of PCL:COL that can be used to fabricate electrospun sheets using the drum collector.**

| **Mass ratio** | | **Electrospinning condition** | | | |
|---|---|---|---|---|---|
| **Mass PCL** | **Mass Collagen** | **Rounds Per Minute (RPM)** | **Duration (h)** | **Voltage (kV)** | **Flow rate (mL/h)** |
| 77 | 23 | 1200 | 8 | 10 | 0.3 |
| 77 | 23 | 1200 | 10 | 10 | 0.3 |
| 68 | 32 | 1200 | 10 | 10 | 0.3 |
| 60 | 40 | 1900 | 3 | 14 | 0.3 |
| 60 | 40 | 1500 | 1 | 14 | 0.3 |
| 60 | 40 | 1900 | 1 | 14 | 0.3 |
| 60 | 40 | 1900 | 2 | 14 | 0.3 |
| 60 | 40 | 1900 | 3 | 14 | 0.3 |
| 51 | 49 | 1200 | 10 | 10 | 0.3 |
| 50 | 50 | 2500 | 2 | 20 | 0.3 |
| 49 | 51 | 1200 | 8 | 10 | 0.3 |
| 49 | 51 | 1200 | 9 | 10 | 0.3 |
| 49 | 51 | 1200 | 10 | 10 | 0.3 |
| 49 | 51 | 1200 | 10 | 11 | 0.3 |
| 49 | 51 | 1200 | 10 | 12 | 0.3 |
| 49 | 51 | 1200 | 12 | 10 | 0.3 |
| 49 | 51 | 2000 | 10 | 10 | 0.3 |

For the latter, the electrospun fibers were collected in between the rods of a grid collector (Inovenso, Turkey) situated about 18 cm from the tip of the needle after 1-5 minutes of electrospinning, and formed into scaffolds by pushing a round glass cover slip (D: 18mm, Jena Bioscience, Germany) in the same orientation through the rods where fibers are present. A 200-layer scaffold was formed when the cover slip was pushed through a total of 200 times (Table 1B).

**Table 1B: List of mass ratios of PCL:COL that can be used to fabricate electrospun sheets using the grid collector.**

| **Mass ratio** | | **Electrospinning condition** | |
|---|---|---|---|
| **Mass PCL** | **Mass Collagen** | **Voltage (kV)** | **Flow rate (mL/h)** |
| 77 | 23 | 10 | 0.3 |
| 77 | 23 | 11 | 0.3 |
| 77 | 23 | 12 | 0.3 |
| 77 | 23 | 13 | 0.3 |
| 77 | 23 | 14 | 0.3 |
| 77 | 23 | 18 | 0.3 |
| 68 | 32 | 10 | 0.3 |
| 68 | 32 | 11 | 0.3 |
| 68 | 32 | 12 | 0.3 |
| 68 | 32 | 13 | 0.3 |
| 68 | 32 | 14 | 0.3 |
| 51 | 49 | 18 | 0.3 |
| 50 | 50 | 14 | 0.3 |
| 42 | 58 | 18 | 0.3 |

### Post-spinning treatments:

Crosslinking: Crosslinking forms chemical bonds among collagen structures that result in fibrous scaffolds' strength and flexibility that feature within the range of natural human cornea. For EDC/NHS crosslinking, 1-Ethyl-3-(3-dimethylaminopropyl) carbodiimide (EDC) (Thermo Scientific, Singapore) and N-Hydroxysuccinimide (NHS) (Thermo Scientific, Singapore) are diluted in reagent ethanol to form a 200 mM crosslinking solution. The PCL-COL scaffolds are then immersed in the crosslinking solution for 5 hours. After which, scaffolds are washed in normal saline solution (NSS) (B Braun, Singapore) for 18 hours (FIG. 2A). If scaffolds are electrospun onto a sheet of aluminum foil on the drum collector, they are peeled from the aluminum foil after 18 hours of soaking in NSS and dried. The scaffolds are then sent for oxygen plasma treatment. They may be crosslinked a second time for 5 hours after treatment with oxygen plasma.

Other crosslinkers that serve to strengthen the scaffolds include: Glutaraldehyde vapor, UV light, Riboflavin, genipin, and paraformaldehyde. For glutaraldehyde vapor cross-linking, glutaraldehyde, 25% v/v (Acros Organics, Singapore) is diluted in distilled water to form a 20% v/v solution. The solution is left to evaporate in a sealed beaker containing electrospun PCL-COL scaffolds, for 8 hours at 25°C. For UV light and Riboflavin, crosslinking is conducted using the Dresden protocol: 0.1% (w/v) riboflavin-5-sulfate is dissolved in 20% (w/v) dextran solution. Scaffolds are then immersed in the solution for 30 minutes before they are placed under a UVA lamp (370 nm) for 30 minutes with drops of solution added every 5 minutes. For Genipin and paraformaldehyde crosslinking, scaffolds are immersed in 0.1% (w/v) Genipin in reagent alcohol for 2 hours and 4% v/v Paraformaldehyde solution for 30 minutes respectively at 25°C (Table 2A).

FIG. 2A illustrates a schematic diagram 200A showing the crosslinking process, in accordance with at least one embodiment.

**Table 2A: Crosslinking conditions for PCL-collagen scaffolds**

| **Chemical** | **Concentration** | **Solvent** | **Time** |
|---|---|---|---|
| Glutaraldehyde | Vapor, 20% v/v | DI Water | 18 hours |
| Glutaraldehyde | Solution, 0.2% v/v | DI Water | 1 hour |
| Glutaraldehyde | Solution, 0.2% v/v | DI Water | 3.5 hours |
| Glutaraldehyde | Solution, 0.2% v/v | DI Water | 18 hours |
| Glutaraldehyde | Solution, 0.2% v/v | DI Water | 5 minutes |
| Glutaraldehyde | Solution, 0.2% v/v | DI Water | 15 minutes |
| Glutaraldehyde | Solution, 0.2% v/v | DI Water | 30 minutes |
| EDC/NHS | 200 mM EDC, 200 mM NHS | Reagent ethanol | 5 hours |
| EDC/NHS | 200 mM EDC, 200 mM NHS | Reagent ethanol | 18 hours |
| UV/Riboflavin | 0.1% w/v riboflavin-5-sulfate for 30 min, UVA lamp (370 nm) for 30 min, with drops of solution added every 5 min | Dextran solution | - |
| Genipin | 0.10% w/v | Reagent ethanol | 2 hours |
| Paraformaldehyde | 4% v/v | - | 30 minutes |

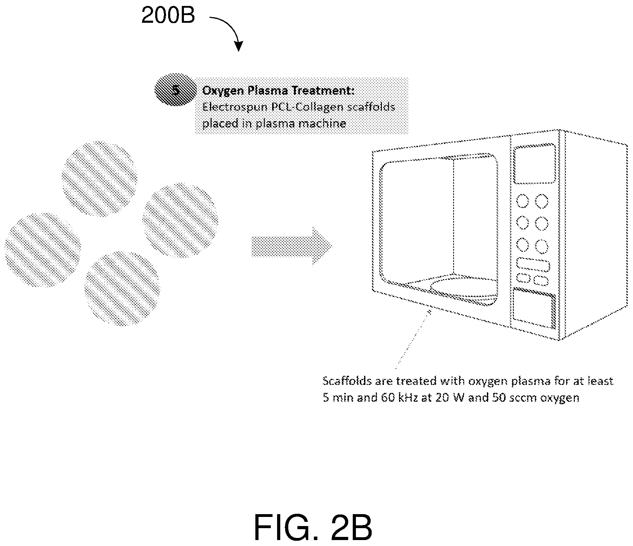

Plasma Treatment: The scaffolds also feature hydrophilic wettability, allowing them to attach to the host's existing cornea, as well as transparency, allowing them to function effectively like natural cornea. These features are achieved by oxygen plasma treatment. Plasma treatment was conducted on electrospun PCL-collagen scaffolds using the COVANCE Surface Plasma Treatment System (FIG. 2B) (FEMTO Science, Korea). Glow plasma was created with 50 sccm oxygen gas at a power of 20 W, with the frequency typically at 70 kHz, but varied to from 60-80 kHz. The scaffolds are placed under plasma for at least 5 minutes up to 120 min (Table 2B).

FIG. 2B illustrates a schematic diagram 200B showing the plasma treatment process, in accordance with at least one embodiment.

**Table 2B: Table of plasma treatment conditions**

| **Frequency (kHz)** | **Power (W)** | **Duration (min)** | **Oxygen (sccm)** |
|---|---|---|---|
| 80 | 20 | 15 | 50 |
| 80 | 20 | 20 | 50 |
| 80 | 20 | 40 | 50 |
| 70 | 20 | 5 | 50 |
| 70 | 20 | 10 | 50 |
| 70 | 20 | 15 | 50 |
| 70 | 20 | 20 | 50 |
| 70 | 20 | 30 | 50 |
| 70 | 20 | 40 | 50 |
| 70 | 20 | 60 | 50 |
| 70 | 20 | 75 | 50 |
| 70 | 20 | 90 | 50 |
| 70 | 20 | 120 | 50 |
| 60 | 20 | 40 | 50 |

Stacking (more than one layer of scaffolds): Gelatin powder (Sigma-Aldrich, Singapore) was dissolved in DI H₂O to form a gelatin solution of 2% w/v. Single-layer PCL-COL scaffolds, having undergone plasma treatment and EDC/NHS crosslinking previously, were immersed in the 2% w/v gelatin solution for 30 minutes. After which, single layers are stacked on top of each other until a 4-layer construct is formed. The 4-layer scaffolds are crosslinked with EDC/NHS for 18 hours before being washed with DI H₂O.

### Physical Characterization

Scanning Electron Microscopy: The surface morphology of PCL-COL electrospun scaffolds was examined with Scanning Electron Microscopy (JEOL JSM 6360, Japan), where scaffolds were sputter-coated with platinum and observed at an accelerating voltage of 5 kV. Images were then captured. Three scaffolds were examined, for a total of three repeats. For each scaffold, the image analysis software, ImageJ (National Institutes of Health, USA), was used to determine the average fiber diameters and standard deviation from 100 measurements.

### Chemical Characterization

Fourier-transform infrared spectroscopy (FTIR): Chemical analysis of the PCL-COL electrospun scaffolds was performed by FTIR spectroscopy (PerkinElmer Spectrum 100, USA) over a range of 4000 to 650 cm⁻¹. In addition, FTIR spectroscopy was also performed for an electrospun pure PCL scaffold and an electrospun pure collagen scaffold to act as references.

### Mechanical Characterization

The mechanical properties of PCL-collagen electrospun scaffolds are investigated using the following steps: scaffolds were cut into 40 x 10 mm strips instead of the circular shapes. Post-spinning treatments still apply. The scaffold strips were then loaded on the Instron 3340 machine (Agency for Science, Technology and Research, Singapore), with a 50 N load cell at the crosshead speed of 1 mm/min, in accordance with ASTM D882-02. Young's modulus (elasticity modulus) was then determined from the slope of the Load-Extension curve obtained, and the Ultimate Tensile strength was determined at the highest tensile strength value before the breakpoint.

### Light Transmittance

To measure the transmittance of electrospun PCL-COL scaffolds, scaffolds were transferred onto glass cover slips (Jena Bioscience, Germany) for support. The scaffolds on glass slips are subsequently placed in wells of a 24-well plate (Thermo Fisher, Singapore) and immersed in NSS for 10 minutes before being placed under the Spark Multimode Microplate Reader (Tecan Trading AG, Switzerland). The microplate reader was used to measure the absorbance of the scaffolds under the wavelengths between 400 to 700 nm. In addition, the absorbance values for a glass cover slip and a PCL-COL scaffold not immersed in PBS were also measured. The transmittance values were then calculated by the equation: T(%) = 10^{(2-A)}, where A and T are absorbance and transmittance, respectively.

### Results:

Corneal regeneration composition should contain an aligned fiber layer of biocompatible polycaprolactone and collagen.

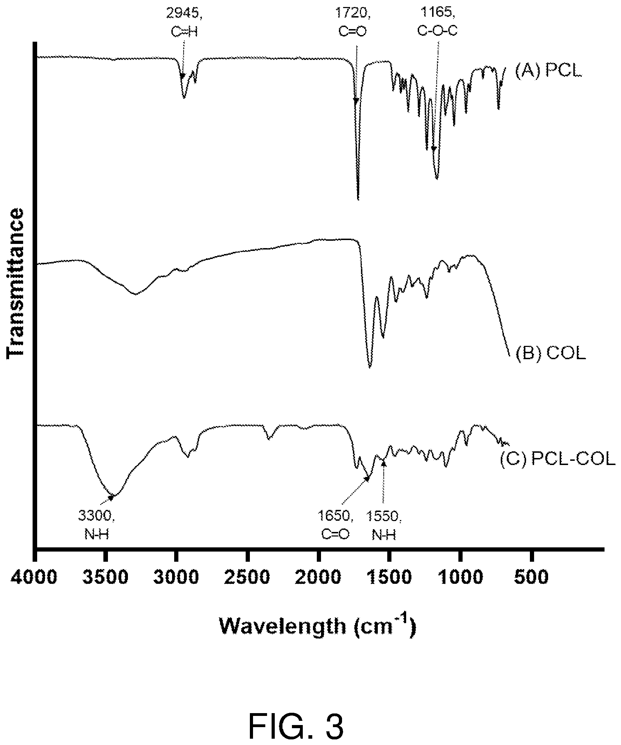

Electrospinning a mixed solution of collagen and biocompatible PCL can produce a scaffold containing both collagen and PCL. FIG. 3(A) shows the FTIR spectrum of electrospun pure PCL scaffold, with unique peaks at 2945, 1720, and 1165 cm⁻¹. FIG. 3(B) shows the FTIR spectrum of the electrospun pure collagen scaffold, with unique peaks at 3300, 1650, and 1550 cm⁻¹. When both solutions are combined, the FTIR graph of the resultant electrospun scaffold contains unique peaks found in the PCL reference as well as the atelocollagen reference, proving the presence of both polymers in the scaffold, as shown in FIG. 3(C).

FIG. 3 illustrates (A) FTIR spectrum of electrospun pure PCL scaffold, with unique peaks at 2945, 1720 and 1165 cm⁻¹; (B) FTIR spectrum of electrospun pure collagen scaffold, with unique peaks at 3300, 1650, and 1550 cm⁻¹; (C) FTIR spectrum of PCL-collagen scaffold, with unique peaks at 2945, 1720 and 1165 cm⁻¹ matching PCL, and 3300, 1650 and 1550 cm⁻¹ matching collagen.

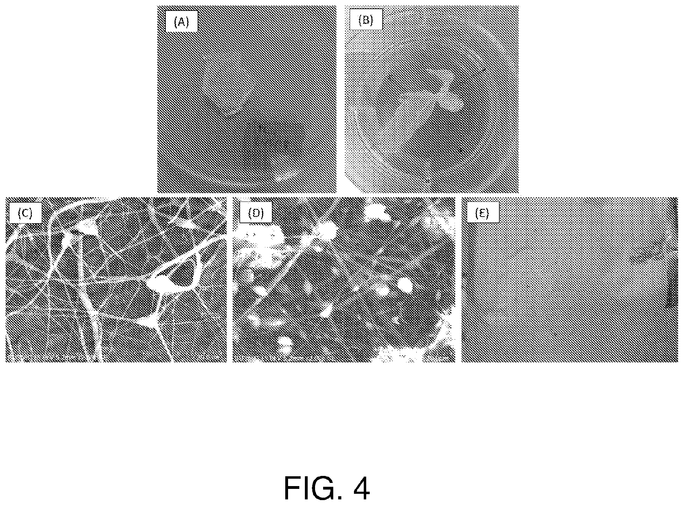

PCL-Atelocollagen mixture is a more advantageous solution than pure PCL solution (FIG. 4A) in electrospinning to obtain transparent and hydrophilic scaffolds, and more advantageous than pure collagen solution (FIG. 4C) in electrospinning to obtain aligned fibers without beads. The mass ratio of PCL to collagen to form electrospun sheets using a grid collector is from 77:23 to 42:58 and using a drum collector is from 77:23 to 49:51. When PCL proportion falls below the range, the fibers will be generated as uneven fibers that feature droplets or beads, as shown in FIGS. 4D and 4E. When the PCL proportion is above the range, the scaffold will turn out opaque and hydrophobic rather than transparent, as shown in FIG. 4B.

FIGS. 4A-4B illustrate various perspective views of the scaffold with a mass ratio of PCL and collagen 100:0 (4A) and 80:20 (4B), which are white, opaque, and relatively hydrophobic, shriveling up in PBS.

FIGS. 4C-4D illustrate various perspective views of 2500x magnification SEM images of the scaffold with the mass ratio of PCL and collagen 0:100 (4C) and 40:60 (4D), which the fibers are not of good quality, with multiple droplets.

FIG. 4E illustrates a perspective view of a poor-quality scaffold produced when the PCL: collagen ratio is 40:60, which is thin, fragile, and full of droplets.

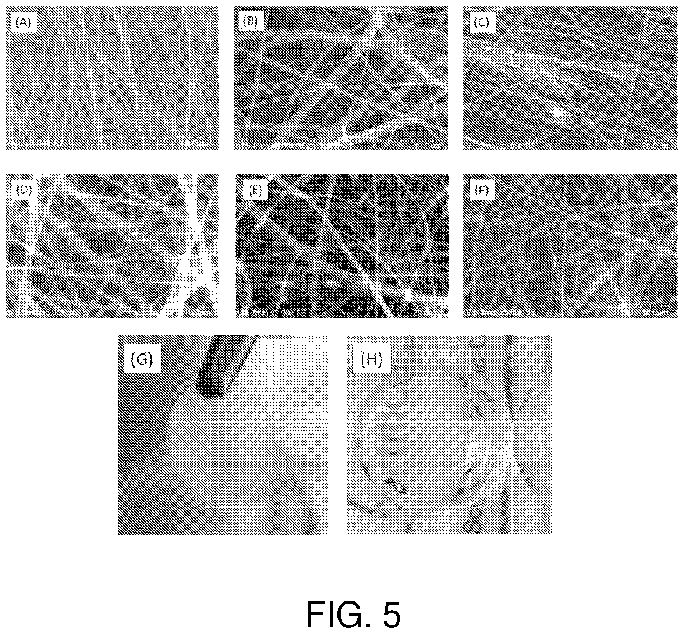

When the mass ratio of PCL to collagen to form electrospun sheets using a grid collector is from 77:23 to 42:58 and using a drum collector is from 77:23 to 49:51, random or aligned fibers with no beads or droplets with collagen and PCL are formed. This is illustrated in FIG. 5A to 5F. Aligned fibers are obtained by rotating the drum collector or collecting in between the grids of the grid collector, while random fibers are obtained when the drum collection is stationary. With aligned fibers and post-fabrication treatments, as will be shown in later parts, scaffolds can reach a good level of transparency, as demonstrated in FIGS. 5G and 5H.

FIG. 5 illustrates perspective views of SEM images of PCL-collagen scaffolds with various mass ratios of PCL: collagen at (A) 77:23, (B) 72:28, (C) 60:40, (D) 51:49, (E) 50:50, and (F) 42:58. Smooth fibers can be obtained. The scaffold having a mass ratio of PCL: collagen at 77:23 electrospun using the grid collector as shown in (G) and the scaffold having a mass ratio of PCL: collagen at 49:51 electrospun using the drum collector as shown in (H) and after EDC/NHS crosslinking show transparency.

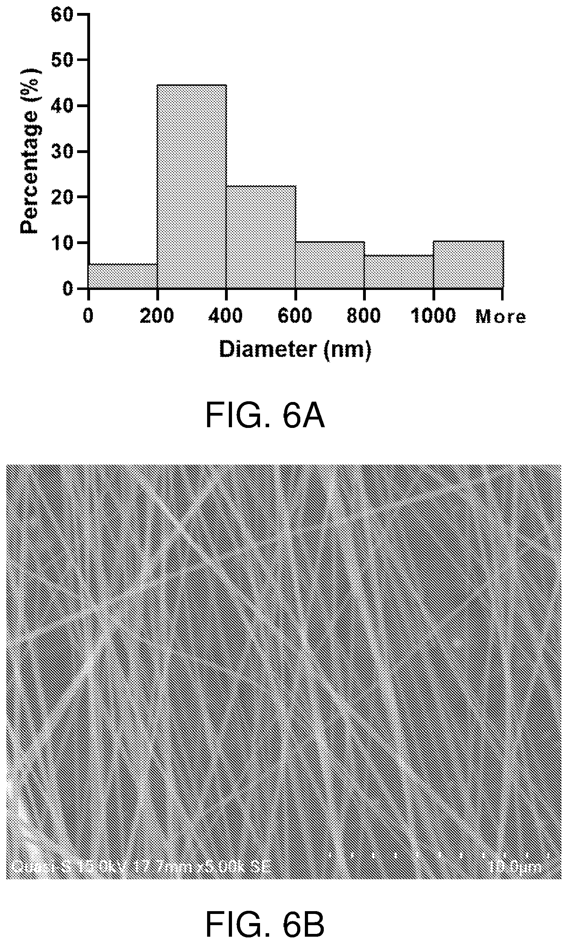

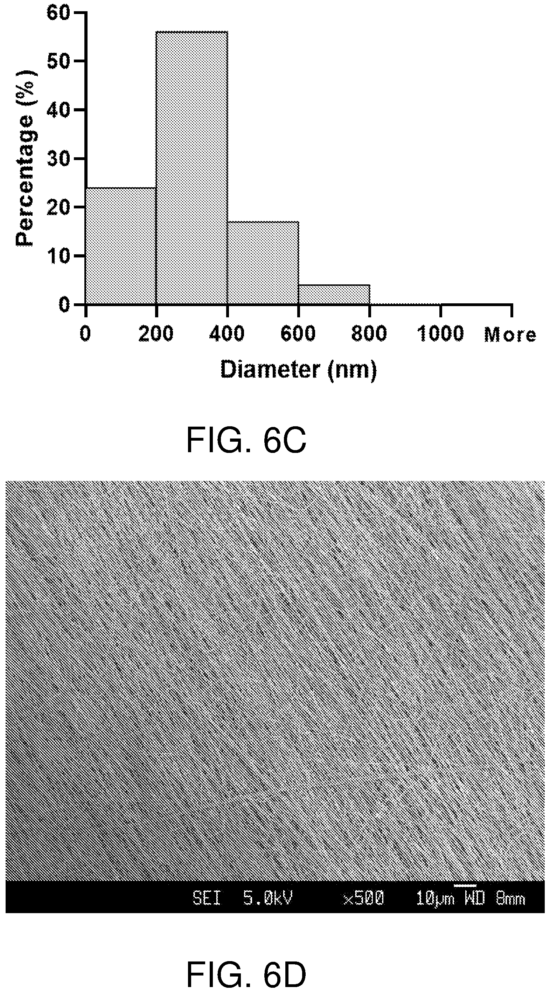

The alignment of electrospun fibers of PCL and collagen collected on aluminum foil was observed using scanning electron microscopy (SEM), as shown in FIG. 6. The fiber diameters of the scaffolds were mixed between 125 nm to 1500 nm. The majority (44.5%) of fiber diameter was at about 300 nm, which is more similar to native human cornea than previous studies of electrospun PCL and collagen scaffolds reported that the fiber diameter was approximately 500 nm.

FIG. 6A illustrates a perspective view of the diameter range histogram of electrospun PCL-collagen scaffolds spun using the grid collector. The mass ratio of PCL and collagen is 77:23, in accordance with at least one embodiment.

FIG. 6B illustrates a perspective view of the SEM image of PCL-collagen scaffolds spun using the grid collector (mass ratio 77:23), in accordance with at least one embodiment.

FIG. 6C illustrates a perspective view of the diameter range histogram of electrospun PCL-collagen scaffolds spun using the drum collector. The mass ratio of PCL and collagen is 77:23, in accordance with at least one embodiment.

FIG. 6D illustrates a perspective view of the SEM image of PCL-collagen scaffolds spun using the drum collector (mass ratio 77:23), in accordance with at least one embodiment.

### Crosslinking the layer of aligned fibers yields scaffolds that are equivalent in strength to the human cornea

Young's modulus of the cornea usually varies widely from several studies which can range between 0.1 and 57 MPa depending on the region of the stroma characterized, donor age, period of storage, and measurement method used. Elasticity properties of explant native corneas were measured and Young's modulus was found to be in the range of 0.3 MPa to 7.0 MPa. The ultimate tensile strength signified the ability to withstand weight loads. In the native cornea, it is measured to be less than or equal 19.1 MPa.

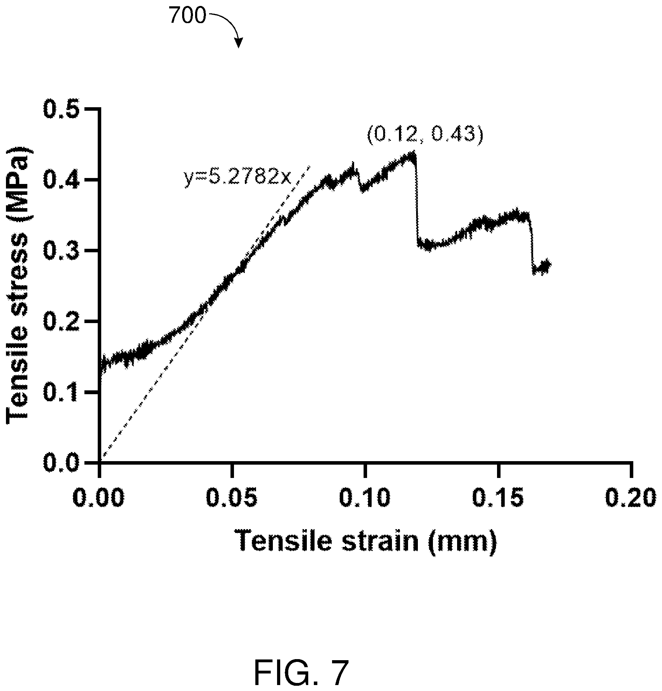

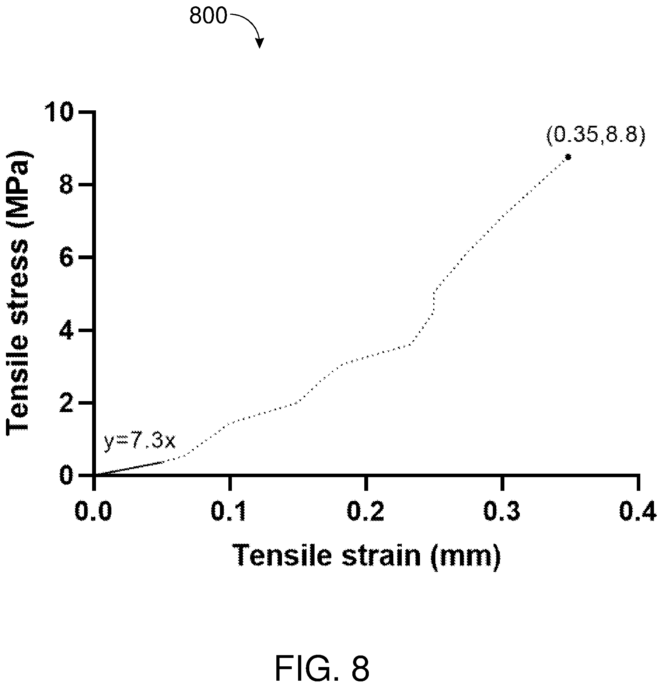

In FIG. 7, when the scaffolds of PCL-collagen at a mass ratio of 49:51 are crosslinked twice in EDC/NHS for 5 hours both before and after plasma treatment, the Young's Modulus obtained is approximately 5.27 MPa and the Ultimate Tensile Strength is approximately 0.43 MPa, within the ranges of the native cornea. This means that the scaffolds have elasticity (measured by Young's Modulus) and the ability to withstand loads (measured by Ultimate Tensile Strength) comparable to the native cornea. This also applies to PCL-collagen scaffolds (mass ratio of 68:32) electrospun with the drum collector and crosslinked by 0.2% v/v glutaraldehyde solution, where Young's Modulus approximately 7.30 MPa and Ultimate Tensile strength approximately 8.80 MPa, as shown in FIG. 8.

FIG. 7 illustrates a graphical representation 700 of a stress-strain curve of the scaffolds of PCL-collagen at a mass ratio of 49:51 which are crosslinked twice in EDC/NHS for 5 hours both before and after plasma treatment, in accordance with at least one embodiment.

FIG. 8 illustrates a graphical representation 800 of stress-strain graphs of PCL-collagen scaffolds (mass ratio of 68:32) electrospun with the drum collector and crosslinked by 0.2% glutaraldehyde solution. Young's Modulus and Ultimate Tensile strength are calculated for each crosslink condition. Young's Modulus approximately 7.30 MPa. Ultimate Tensile strength approximately 8.80 MPa.

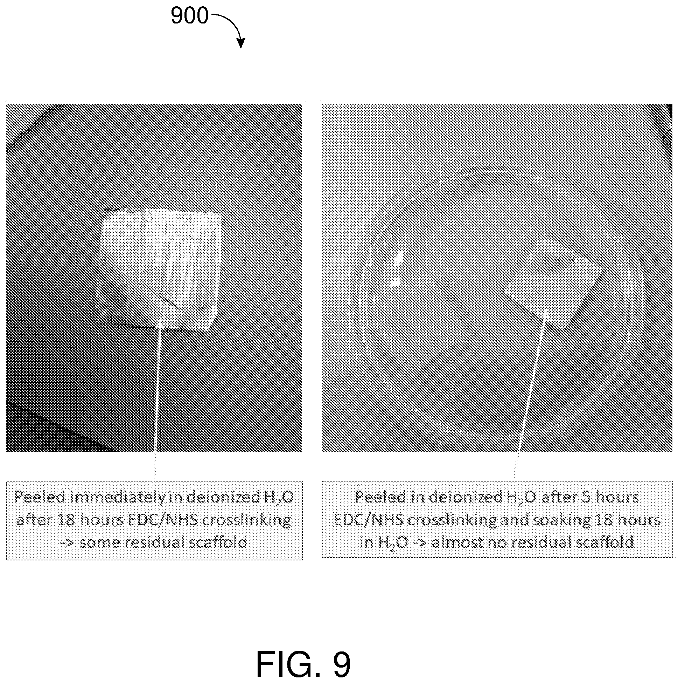

After crosslinking electrospun scaffolds with EDC/NHS for 5 hours, soaking the washed scaffold in NSS for 18 hours before peeling off the scaffold from the aluminum foil gives more uniform scaffolds. Without soaking for 18 hours before peeling the scaffolds can be torn and lose their uniformity as sheets. The electrospun fibrous layer was easily peeled from the foil after 18 hours of soaking in water or NSS after crosslinking with EDC/NHS for 5 hours, leaving no residual scaffold behind on the foil and giving a uniform scaffold. This is a better way of producing a uniform scaffold, as shown in FIG. 9.

FIG. 9 illustrates a perspective view 900 of a comparison of peeling the scaffolds after 18 hours vs. 5 hours of EDC/NHS crosslinking at 25 °C, in accordance with at least one embodiment.

Plasma treatment using the flow rate of Oxygen gas at 50 sccm at the power of 20 W with the frequency of 70 kHz for 20 minutes to 119 minutes improves the transparency of scaffolds.

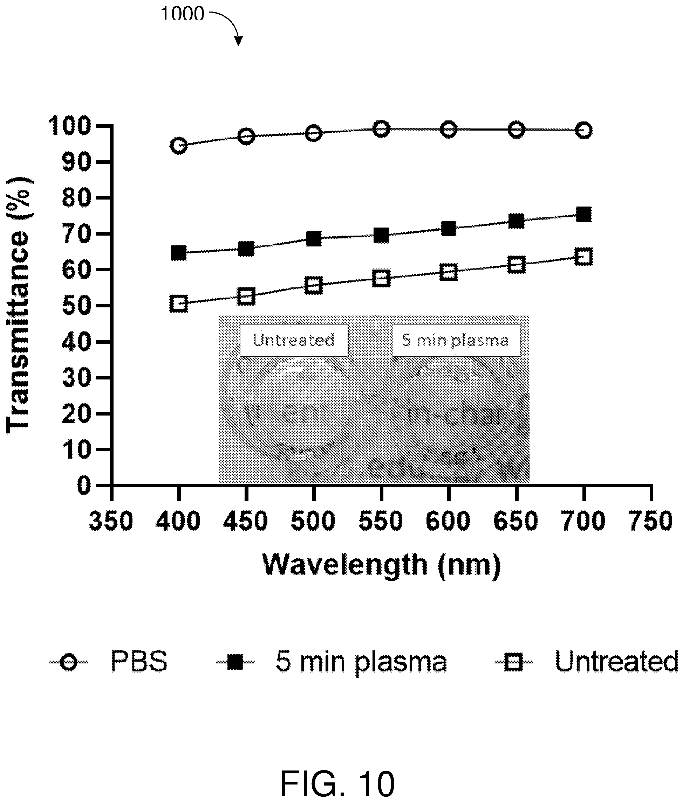

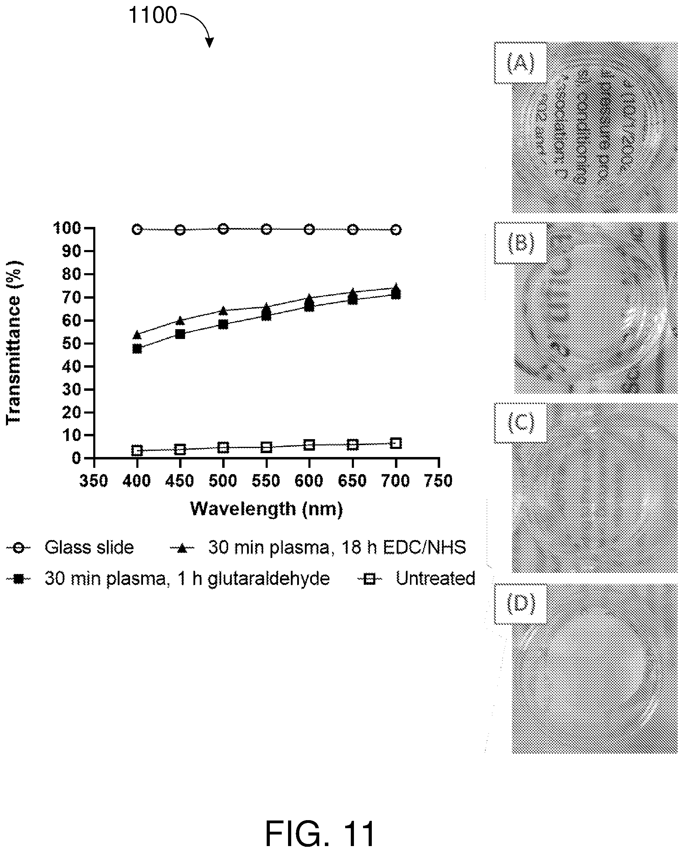

Plasma treatment with various frequency and duration (Table 3 and 4) was used to improve transparency of scaffolds, however, with this specific condition of our work only oxygen gas was used and at least 5 min the scaffolds must be treated. As shown in FIG. 10, 5 min of plasma treatment can improve scaffold transmittance to around 64.7±0.00% to 75.3±0.00% for 300-layer scaffolds spun with the grid collector, compared to untreated scaffolds with around 50% to 60%. The increase in transmittance after plasma treatment also applies to scaffolds spun onto the rotating drum collector, from 3.3±0.00% to 6.5±0.00% without plasma to 53.9±0.00% to 74.2±0.00% with plasma, as shown in FIG. 11.

**Table 3: Transparency outcomes for scaffolds spun using the grid collector under various PCL: Collagen mass ratios and plasma treatment conditions. Plasma treatment is set with the flow rate of Oxygen gas at 50 sccm at the power of 20 W.**

| **Electrospinning** | | | **Plasma treatment** | | **Transmittance** | **Disintegration** |
|---|---|---|---|---|---|---|
| **Mass PCL** | **Mass Collagen** | **No. of layers** | **Frequency (kHz)** | **Duration (min)** | **% (from 400 nm to 700 nm)** | |
| 77 | 23 | 200 | 60 | 40 | 55.5±0.00 - 68.1±0.00 | No |
| 77 | 23 | 200 | 70 | 40 | 78.8±0.00 - 87.1±0.00 | No |
| 77 | 23 | 200 | 60 | 40 | 55.0±0.38 - 65.8±0.04 | No |
| 77 | 23 | 200 | 70 | 40 | 80.0±2.73 - 88.1±1.24 | Yes |
| 77 | 23 | 200 | 80 | 40 | 95.6±4.07 - 96.5±1.94 | Yes |
| 77 | 23 | 200 | 70 | 40 | 71.8±3.04 - 74.1±3.25 | No |
| 77 | 23 | 200 | 70 | 40 | 86.8±9.13 - 89.5±4.07 | No |
| 77 | 23 | 200 | 60 | 40 | 66.5±12.80 - 74.2±10.10 | No |
| 77 | 23 | 200 | 70 | 40 | 87.7±7.84 - 92.3±5.78 | Yes |
| 77 | 23 | 200 | 70 | 40 | 85.1±8.31 - 90.3±5.00 | Yes |
| 77 | 23 | 200 | 70 | 40 | 94.8±2.17 - 96.5±1.53 | Yes |
| 77 | 23 | 200 | 70 | 40 | 91.4±2.98 - 94.6±2.03 | Yes |
| 77 | 23 | 300 | 70 | 30 | 76.1±6.61 - 83.2±3.35 | Yes |
| 77 | 23 | 300 | 70 | 15 | 54.4±8.89 - 68.3±8.35 | No |
| 77 | 23 | 30 | 40 | 5 | 67.9±0.00 - 79.9±0.00 | No |
| 77 | 23 | 30 | 40 | 10 | 79.2±0.00 - 88.0±0.00 | No |
| 77 | 23 | 30 | 40 | 20 | 89.6±0.00 - 94.4±0.00 | No |
| 77 | 23 | 30 | 50 | 5 | 81.8±0.00 - 89.5±0.00 | No |
| 77 | 23 | 30 | 60 | 5 | 81.0±0.00 - 88.9±0.00 | No |
| 77 | 23 | 300 | 70 | 5 | 64.7±0.00 - 75.3±0.00 | No |
| 77 | 23 | 300 | 70 | 10 | 71.6±0.00 - 79.3±0.00 | No |
| 77 | 23 | 300 | 70 | 15 | 69.9±0.00 - 78.6±0.00 | No |

**Table 4: Transparency outcomes for scaffolds spun using the rotating drum (1200 RPM) collector at 10 kV with flow rate of 0.3 ml/h under various PCL: Collagen mass ratios and plasma treatment conditions. Plasma treatment is set with the flow rate of Oxygen gas at 50 sccm at the power of 20 W.**

| **Electrospinning** | | | **Plasma treatment** | | **Transmittance** | **Disintegration** |
|---|---|---|---|---|---|---|
| **Mass PCL** | **Mass Collagen** | **Spinning Duration (h)** | **Frequency (kHz)** | **Duration (min)** | **% (from 400 nm** to **700 nm)** | |
| 68 | 32 | 8 | 70 | 5 | 19.2±2.06 - 43.9±2.54 | No |
| 68 | 32 | 8 | 70 | 10 | 13.8±2.51 - 34.5±3.93 | No |
| 68 | 32 | 8 | 70 | 15 | 12.2±1.97 - 33.6±3.48 | No |
| 68 | 32 | 8 | 70 | 30 | 24.6±0.00 - 52.7±0.00 | No |
| 68 | 32 | 8 | 80 | 15 | 15.6±1.58 - 39.8±1.48 | No |
| 68 | 32 | 8 | 70 | 5 | 26.7±4.48 - 51.1±5.33 | No |
| 68 | 32 | 8 | 70 | 10 | 12.7±0.00 - 35.1±0.00 | No |
| 68 | 32 | 8 | 70 | 15 | 19.1±0.00 - 40.6±0.00 | No |
| 68 | 32 | 8 | 70 | 30 | 30.4±16.10 - 48.9±14.80 | No |
| 68 | 32 | 8 | 80 | 15 | 8.36±0.98 - 27.4±1.66 | No |
| 68 | 32 | 8 | 70 | 5 | 11.7±1.52 - 34.1±2.85 | No |
| 68 | 32 | 8 | 70 | 10 | 20.8±3.54 - 45.7±4.80 | No |
| 68 | 32 | 8 | 70 | 15 | 20.5±0.00 - 46.9±0.00 | No |
| 68 | 32 | 8 | 70 | 30 | 32.2±0.00 - 56.4±0.00 | No |
| 68 | 32 | 8 | 80 | 15 | 16.8±3.93 - 39.9±3.17 | No |
| 68 | 32 | 8 | 70 | 5 | 17.8±2.04 - 41.2±3.27 | No |
| 68 | 32 | 8 | 70 | 10 | 12.8±2.48 - 32.4±3.89 | No |
| 68 | 32 | 8 | 70 | 15 | 11.2±1.71 - 31.4±3.12 | No |
| 68 | 32 | 8 | 70 | 5 | 25.1±5.14 - 48.2±6.51 | No |
| 68 | 32 | 8 | 70 | 10 | 11.7±0.00 - 33.2±0.00 | No |
| 68 | 32 | 8 | 70 | 15 | 16.9±0.00 - 36.7±0.00 | No |
| 68 | 32 | 8 | 70 | 5 | 11.1±1.92 - 32.7±3.89 | No |
| 68 | 32 | 8 | 70 | 10 | 19.9±4.21 - 43.8±6.14 | No |
| 68 | 32 | 8 | 70 | 15 | 18.0±0.00 - 42.4±0.00 | No |
| 59 | 41 | 8 | 70 | 5 | 22.6±3.84 - 46.7±5.08 | No |
| 59 | 41 | 8 | 70 | 10 | 16.4±4.51 - 40.8±6.31 | No |
| 59 | 41 | 8 | 70 | 15 | 20.0±3.62 - 43.2±4.69 | No |
| 59 | 41 | 8 | 70 | 30 | 35.4±2.23 - 60.6±2.88 | No |
| 59 | 41 | 8 | 70 | 5 | 20.1±2.35 - 43.8±1.71 | No |
| 59 | 41 | 8 | 70 | 10 | 24.1±3.33 - 47.6±3.26 | No |
| 59 | 41 | 8 | 70 | 15 | 21.6±6.05 - 48.1±6.45 | No |
| 59 | 41 | 8 | 70 | 30 | 23.1±4.81 - 47.1±4.78 | No |
| 49 | 51 | 8 | 70 | 30 | 69.2±3.25 - 85.6±1.59 | No |
| 49 | 51 | 10 | 70 | 30 | 47.7±0.00 - 71.3±0.00 | No |
| 49 | 51 | 12 | 70 | 30 | 49.0±0.00 - 72.1±0.00 | No |
| 49 | 51 | 8 | 70 | 30 | 61.8±0.00 - 81.9±0.00 | No |
| 49 | 51 | 10 | 70 | 30 | 53.9±0.00 - 74.2±0.00 | No |
| 49 | 51 | 12 | 70 | 30 | 35.0±7.11 - 62.3±4.73 | No |
| 49 | 51 | 8 | 70 | 30 | 61.9±1.53 - 82.0±0.23 | No |
| 49 | 51 | 10 | 70 | 30 | 39.2±0.00 - 65.5±0.00 | No |
| 49 | 51 | 12 | 70 | 30 | 27.4±3.35 - 54.6±2.37 | No |
| 49 | 51 | 9 | 70 | 30 | 53.3±3.12 - 75.1±1.75 | No |
| 49 | 51 | 9 | 70 | 30 | 26.8±3.06 - 56.1±2.58 | No |
| 49 | 51 | 10 | 70 | 30 | 47.7±0.00 - 71.3±0.00 | No |
| 49 | 51 | 10 | 70 | 30 | 56.2±0.00 - 75.6±0.00 | No |
| 49 | 51 | 10 | 70 | 60 | 27.8±1.24 - 56.5±1.16 | No |
| 49 | 51 | 10 | 70 | 60 | 55.6±0.00 - 75.2±0.00 | No |
| 49 | 51 | 10 | 70 | 60 | 46.5±0.00 - 72.1±0.00 | No |
| 49 | 51 | 10 | 70 | 60 | 47.7±0.00 - 70.1±0.00 | No |
| 49 | 51 | 10 | 70 | 30 | 53.9±0.00 - 74.2±0.00 | No |
| 49 | 51 | 10 | 70 | 60 | 57.5±2.20 - 77.5±0.79 | No |
| 49 | 51 | 10 | 70 | 60 | 41.4±2.01 - 65.1±1.85 | No |
| 49 | 51 | 10 | 70 | 60 | 72.6±1.26 - 86.6±1.13 | No |
| 49 | 51 | 10 | 70 | 60 | 66.8±7.68 - 79.8±4.18 | No |
| 49 | 51 | 10 | 70 | 75 | 64.3±3.22 - 82.2%±1.23 | No |
| 49 | 51 | 10 | 70 | 90 | 71.1±4.97 - 86.2±2.71% | No |
| 49 | 51 | 10 | 70 | 120 | 83.4±1.82- 91.9±0.76% | Yes |

FIG. 10 illustrates a graphical representation 1000 of a graph of transmittance over wavelength for 300-layer scaffolds spun with the grid collector. 5 min of electrospinning can improve scaffold transmittance to around 64.7±0.00% to 75.3±0.00%, compared to untreated scaffold with around 50% to 60% transmittance.

FIG. 11 illustrates a graphical representation 1100 of the effect of plasma treatment on the light transmittance of PCL-collagen scaffolds electrospun with the drum collector (mass ratio 77:23). In the Left, a Graph of percentage transmittance of various scaffolds soaked in PBS with different crosslinking conditions for the ultraviolet-visible spectrum is provided. In the Right, Digital photographs show optical transparency of scaffolds, corresponding to the graph. Transmittance values for each condition are: 98.5±0.59% to 98.8±0.37% (glass slide); 53.9±0.00% to 74.2±0.00% (30 min plasma, 18 hours EDC/NHS crosslinking); 47.7±0.00% to 71.3±0.00% (30 min plasma, an hour glutaraldehyde crosslinking); and 3.33±0.00% to 6.49±0.00% (untreated). The control (glass coverslips) showed a maximum transmittance of 98.8±0.37%.

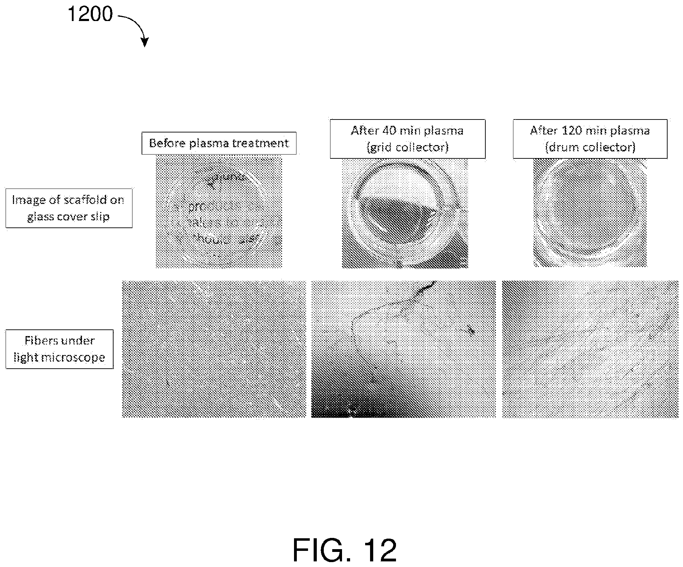

Table 3 also shows that scaffolds spun using the grid collector tend to disintegrate when exposed to 40 min of plasma due to their thinness and fragility, despite their higher transparency. We also found the optimal duration of 30 minutes of oxygen plasma treatment induced the highest transparency with no disintegration of the scaffolds themselves. While 80 kHz and 40 min of plasma exposure give a higher transmittance value, the disintegration of scaffolds often occurs due to the harsh conditions, as shown in FIG. 12.

FIG. 12 illustrates a perspective view 1200 of scaffolds spun on a grid collector and collected on a glass cover slip, before and after plasma treatment with conditions 20 W, 80 kHz, and 40 minutes. After plasma treatment, viewing under a light microscope, fibers can be seen disintegrating. Holes can be seen on the physical surface of the scaffold.

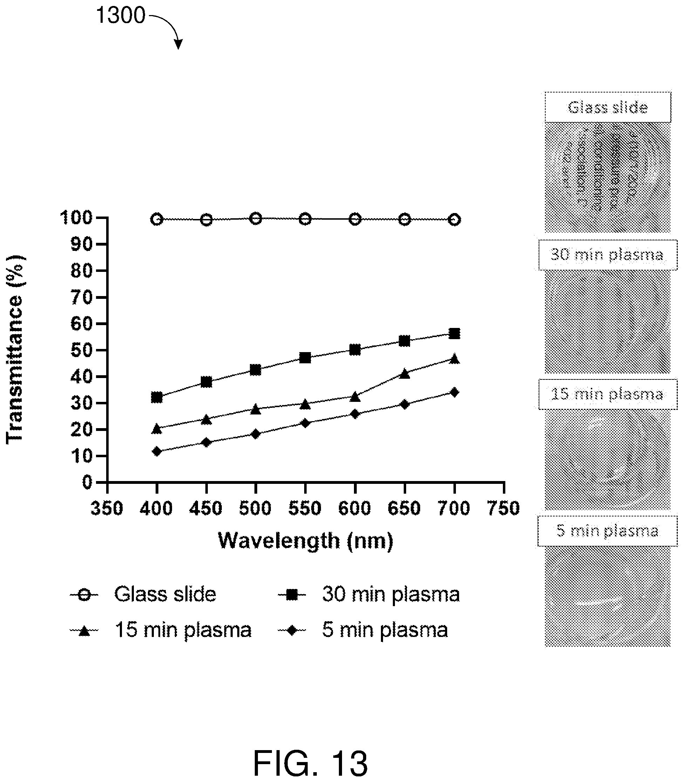

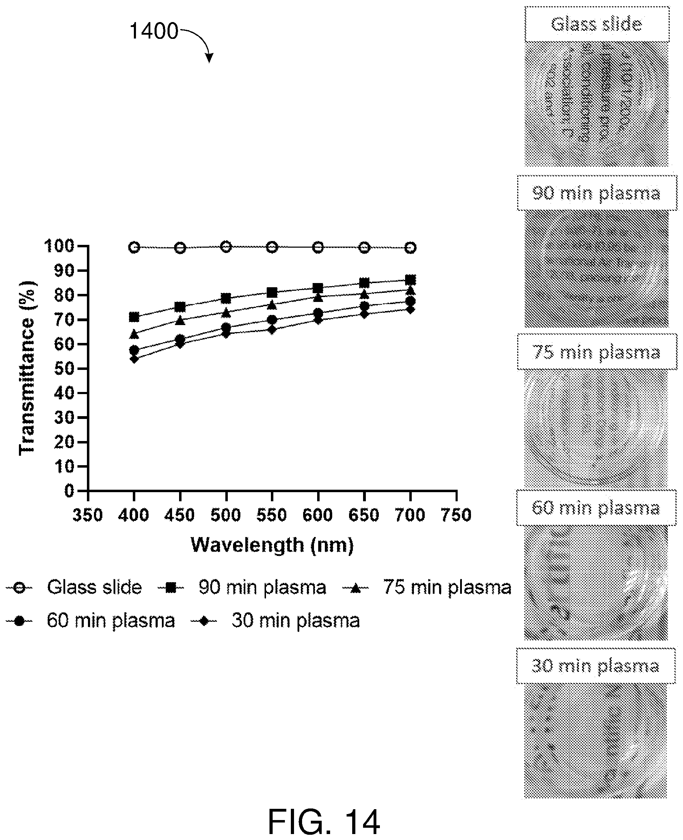

Scaffolds spun with the drum collector are less fragile and are hence not as susceptible to disintegration even with the 60-min plasma treatment. FIG. 13 shows the effect of plasma treatment duration on the transparency of scaffolds (PCL-collagen mass ratio 68:32, non-crosslinked, spun with the rotating drum collector). Plasma machine power and frequency were set at 20 W and 70 kHz respectively. From 5 minutes of plasma treatment to 30 minutes, transmittance increases from 11.7±1.52 to 34.1±2.85 to 32.2±0.00 to 56.4±0.00%. FIG. 14 further illustrates this for PCL-collagen scaffolds having a mass ratio of 49:51, crosslinked with EDC/NHS and spun with the rotating drum collector, where an increase in plasma duration from 30 minutes to 60 minutes increases transmittance from 53.9±0.00 to 74.2±0.00% to 57.5±2.20 to 77.5±0.79%. A slight increase in transparency of the scaffolds was observed from the scaffolds with 60-min plasma treatment.

FIG. 13 illustrates a graphical representation 1300 of the effect of plasma duration on the light transmittance of non-crosslinked PCL-collagen scaffolds electrospun with the drum collector (mass ratio 68:32). In the Left, a Graph of percentage transmittance of various scaffolds after different plasma durations is provided. In the Right, Digital photographs show optical transparency of scaffolds, corresponding to the graph. Transmittance values for each condition are: 98.5±0.59% to 98.8±0.37% (glass slide); 32.2±0.00% to 56.4% (30 min); 20.5% to 46.9% (15 min); and 11.7±1.52% to 34.1±2.85% (5 min).

FIG. 14 illustrates a graphical representation 1400 of the effect of plasma duration on the light transmittance of EDC/NHS cross-linked PCL-collagen scaffolds electrospun with the drum collector (mass ratio 49:51). In the Left, a Graph of percentage transmittance of various scaffolds after different plasma durations is provided. In the Right, Digital photographs show optical transparency of scaffolds, corresponding to the graph. Transmittance values for each condition are: 98.5±0.591% to 98.8±0.374% (glass slide); 64.3±3.22% to 82.2%±1.23% (75 min); 57.5±2.20% to 77.5±0.79% (60 min); and 53.9±0.00 to 74.2±0.00% (30 min).

Plasma treatment, soaking, cross-linking, and washing for a second time increases both scaffold transparency and tensile strength.

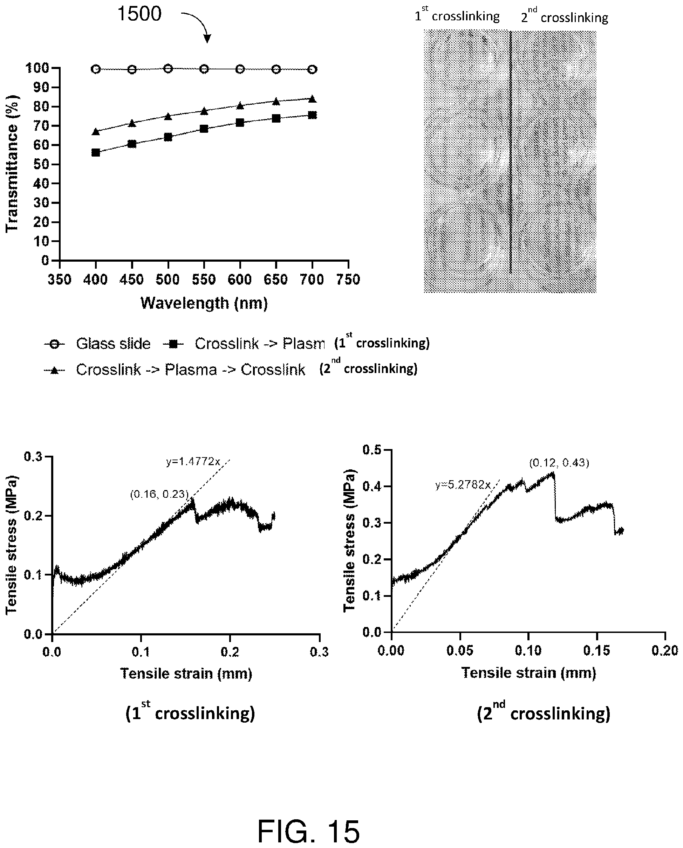

After PCL-collagen scaffolds electrospun with the drum collector (mass ratio 49:51) are crosslinked a second time after plasma treatment in EDC/NHS for 5 hours, the transparency of scaffolds improved by 12.7% (FIG. 15, top). FIG. 15 at the top shows that scaffolds that underwent crosslinking with EDC/NHS before and after plasma treatment have a higher transmittance (67.1±0.00 to 84.2±0.00%) than scaffolds that underwent crosslinking with EDC/NHS only before plasma treatment (56.2±0.00% to 75.6±0.00%). Young's modulus (or elastic modulus) improved by 256% from approximately 1.48 MPa to approximately 5.27 MPa, and ultimate tensile strength improved by 87% from approximately 0.23 MPa to approximately 0.43 MPa because of the second crosslinking (Figure 15, bottom, right) after plasma treatment, compared to scaffolds crosslinked only once (Figure 15, bottom, left). This value of Young's Modulus remains in the range of 0.3 MPa to 7.0 MPa and this value of ultimate tensile strength is less than or equal 19.1 MPa, in the range of those of the native cornea.

FIG. 15 illustrates a graphical representation 1500 of transparency (top) and stress-strain curves of unstack scaffolds after the first crosslinking or 1^{st} crosslinking (bottom left) vs. the second crosslinking or 2^{nd} crosslinking (bottom right), in accordance with at least one embodiment.

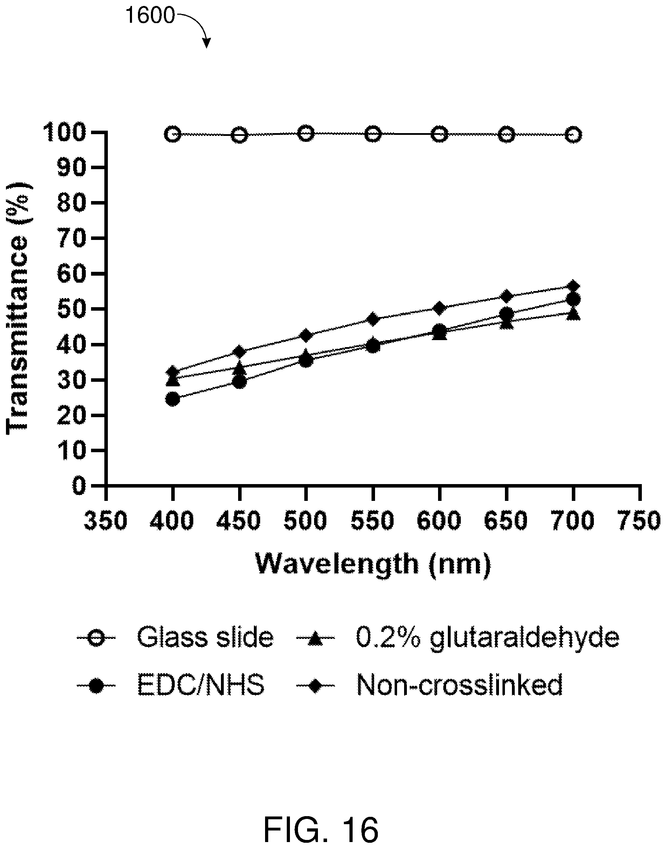

This is already an improvement from the previous data that has shown that scaffolds that underwent crosslinking with EDC/NHS or 0.2% glutaraldehyde solution have a slightly lower transparency than non-plasma treated scaffolds, as shown in FIG. 16. The second EDC/NHS crosslinking conducted according to the present invention improves transparency, and increases the tensile strength and Young's modulus of the scaffolds.

FIG. 16 illustrates a graphical representation 1600 of the effect of plasma treatment on the light transmittance of PCL-collagen scaffolds electrospun with the drum collector (mass ratio 68:32) after plasma treatment at 70 kHz, 20 W, 30 min. Transmittance values for each condition are 30.4±16.10 to 48.9±14.80% (glutaraldehyde-crosslinked scaffolds); 24.6±0.00 to 52.7±0.00% (EDC/NHS-crosslinked scaffolds); and 32.2±0.00 to 56.4±0.00% (non-crosslinked scaffolds). The control (glass coverslips) showed a maximum transmittance of 98.8±0.37%.

The resultant electrospun scaffold can be cut into a desired shape, stacked into a multiple-layer construct, and sutured onto the rabbit's eyes.

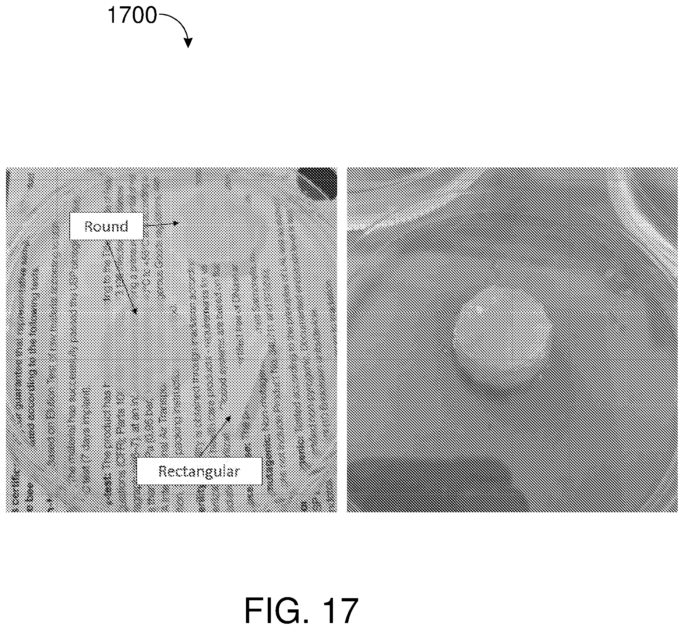

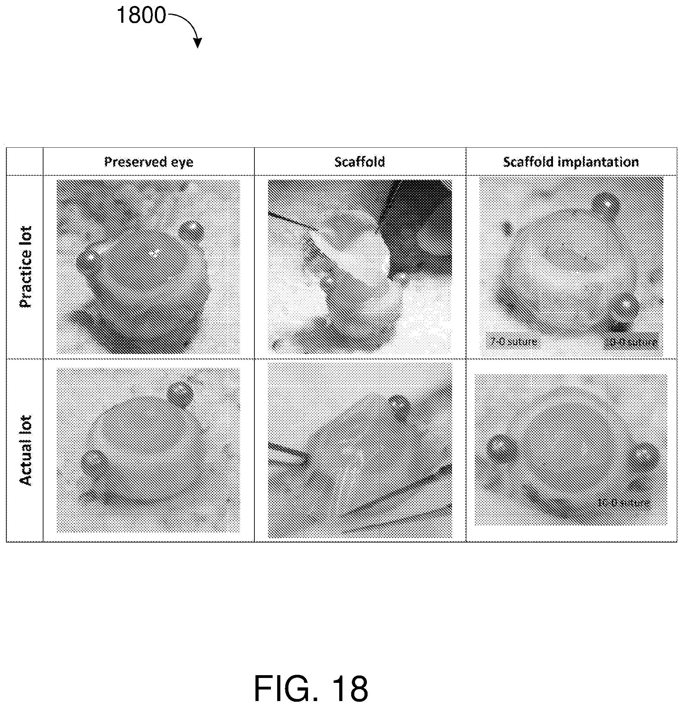

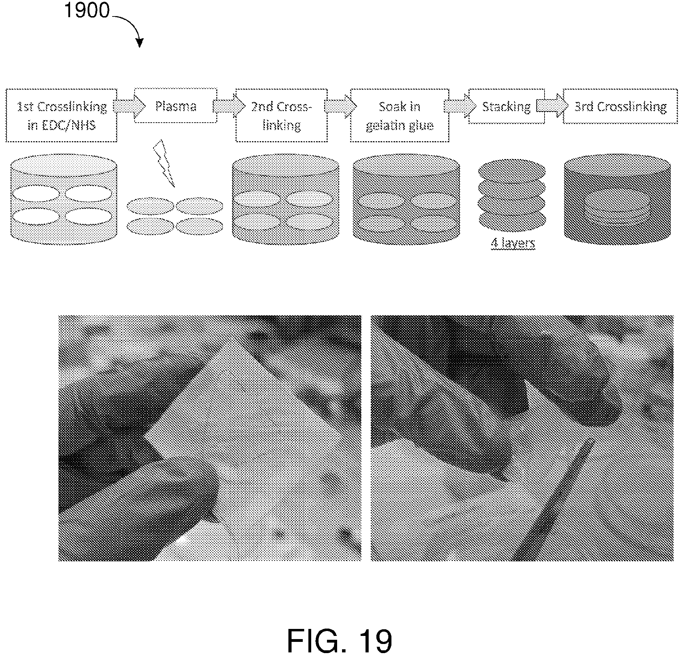

The electrospun films of aligned PCL-collagen fibers were collected on foil for the feasibility of handling and cutting into any shape and at the desired size or diameter, especially a circular shape of eyes. Without these specific techniques, a sheet of aligned straight and even fibers of PCL-collagen cannot be generated in an intact manner. Cutting the scaffold into a circular dome shape that fits the size of the eyes regardless of diameter and species (human, rabbit, pig, etc.) can be shown in FIG. 17 for human eyes and FIG. 18 to be fitted into the wound of rabbit eyes. This cutting also allows for subsequent stacking of the sheets to form thicker and stronger scaffolds as shown in FIG. 19 and to mimic the structure of corneal stroma.

FIG. 17 illustrates a perspective view 1700 of scaffolds according to the present invention that can be cut into all kinds of shapes, like round and rectangular ones as shown in the left. Hence, the scaffolds for corneal implantation can be cut out from the aluminum foil they were spun on to fit the size of the cornea. As shown in the right, a scaffold on an agarose dome can be cut into a circular-dome shape to fit the human cornea. The average corneal diameter is 11-12 mm in humans.

FIG. 18 illustrates a view 1800 of various images from an animal study trial on the preserved rabbit. The scaffolds can be trimmed to fit the size of rabbit eyes (the wound diameter is 6.5 mm). "Practice lot" represents the stacked 5-layer scaffolds and "the actual lot" represents the stacked 4-layer scaffolds. The stacking process is described in FIG. 19 (top).

FIG. 19 illustrates a perspective view 1900 of the method of stacking 4 layers of electrospun scaffolds to obtain the prototype for animal study. The stacked 4-layer scaffolds can be sutured (bottom-left) and trimmed with scissors (bottom-right), being suitable for implantation in the injured eyes of animals.

The specific optimal conditions are required to fabricate tissue-engineered scaffolds that mimic the human cornea, consisting of PCL-collagen electrospinning, oxygen-plasma treatment, and crosslinking with EDC/NHS solution once before plasma treatment, and once after plasma treatment. These scaffolds contain layers of aligned fibers without droplets or beads, a high amount of light transparency, and are mechanically robust, with elasticity and tensile strength in the range of the human cornea. The scaffolds according to the present invention mimic the stromal layer of the cornea, so they are suitable for a potential stromal replacement.

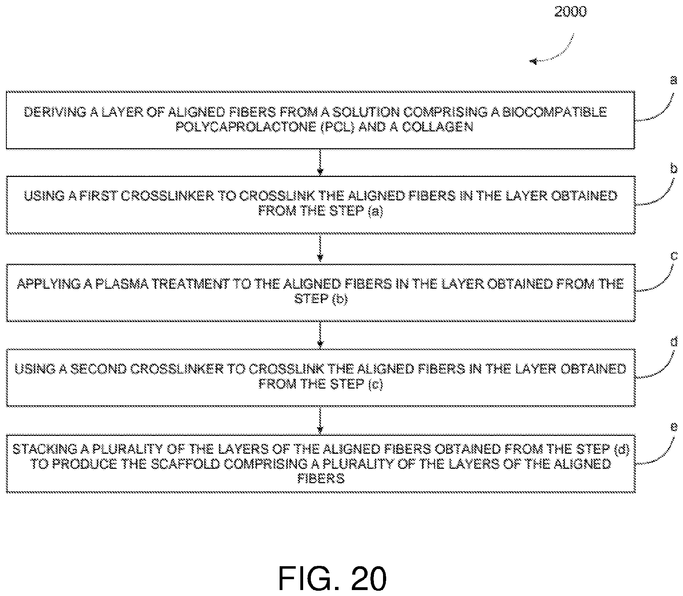

FIG. 20 is a flowchart of a method 2000 for producing a scaffold for corneal regeneration, in accordance with one or more example embodiments. The method comprising a step (a) of deriving a layer of aligned fibers from a solution comprising a biocompatible polycaprolactone (PCL) and a collagen. The PCL and collagen have a mass ratio in a range of 77:23 to 42:58. The method further includes a step (b) of using a first crosslinker to crosslink the aligned fibers in the layer obtained from the step (a). The method further includes a step (c) of applying a plasma treatment to the aligned fibers in the layer obtained from the step (b). The method further includes a step (d) using a second crosslinker to crosslink the aligned fibers in the layer obtained from the step (c). The method further includes a step (e) of stacking a plurality of the layers of the aligned fibers obtained from the step (d) to produce the scaffold comprising a plurality of the layers of the aligned fibers.

In an embodiment, the layer of the aligned fibers has a thickness that is preferably from 15 to 20 µm. In an embodiment, the scaffold preferably comprises four layers of the aligned fibers. In an embodiment, the first crosslinker is selected from the group consisting of 1-Ethyl-3-(3-dimethylaminopropyl) carbodiimide and N-Hydroxysuccinimide (EDC/NHS), Glutaraldehyde, Riboflavin, Genipin, Paraformaldehyde, and any combination thereof. In an embodiment, the first crosslinker is preferably 1-Ethyl-3-(3-dimethylaminopropyl) carbodiimide and N-Hydroxysuccinimide (EDC/NHS). In an embodiment, the second crosslinker is selected from the group consisting of 1-Ethyl-3-(3-dimethylaminopropyl) carbodiimide and N-Hydroxysuccinimide (EDC/NHS), Glutaraldehyde, Riboflavin, Genipin, Paraformaldehyde, and any combination thereof. In an embodiment, the second crosslinker is preferably 1-Ethyl-3-(3-dimethylaminopropyl) carbodiimide and N-Hydroxysuccinimide (EDC/NHS). In an embodiment, the plasma treatment is carried out with an oxygen gas stream at a flow rate of 50 standard cubic centimeters per minute (sccm) and for 20 to 119 minutes.

In an embodiment, the scaffold has various properties that include but are not limited to a visible light transmittance in the range of 50 to 95%; a tensile strength less than or equal to 19.1 MPa; and an elasticity modulus in the range of 1 MPa to 34 MPa.

### Lab testing of tensile strength

### First Test:

### Materials and Methods of lab testing of tensile strength

Scaffolds were cut into 40 x 10 mm strips. They are then immersed in the crosslinking solution (EDC/NHS) for the specified duration. After which, they are washed in normal saline solution (NSS) for 18 hours. The scaffolds are then sent for oxygen plasma treatment. The scaffold strips were then loaded on a metal rod secured to the lab bench. Increasing amounts of weights are then attached to the bottom of the scaffolds, and the subsequent increase in length of the scaffolds is measured, until the scaffold breaks. Young's modulus was then determined from the slope of the Load-Extension curve obtained, and the Ultimate Tensile strength was determined at the highest tensile strength value before the breakpoint.

### Results of lab testing of tensile strength

**Table 5: Summary table of all tensile testing data conducted via in-house lab testing. Plasma treatment is set with the flow rate of Oxygen gas at 50 sccm at the power of 20 W.**

| **Mass PCL** | **Mass Col** | **Crosslinking Duration (h)** | **Plasma Frequency (kHz)** | **Plasma Duration (min)** | **Young's Modulus (MPa)** | **Ultimate Tensile Strength (MPa)** |
|---|---|---|---|---|---|---|
| 68 | 32 | 18 | 70 | 15 | 7.30 | 1.44 |
| 68 | 32 | 18 | 70 | 30 | 6.10 | 0.54 |
| 68 | 32 | 18 | 80 | 15 | 7.30 | 3.38 |
| 59 | 41 | 18 | 70 | 5 | 7.30 | 7.10 |
| 59 | 41 | 18 | 70 | 10 | 3.61 | 3.61 |
| 59 | 41 | 18 | 70 | 15 | 5.48 | 4.51 |
| 59 | 41 | 18 | 70 | 30 | 3.05 | 0.54 |
| 49 | 51 | 18 | 70 | 30 | 7.30 | 8.80 |
| 49 | 51 | 18 | 70 | 30 | 5.48 | 5.05 |
| 49 | 51 | 5 | 70 | 30 | 7.30 | 3.07 |

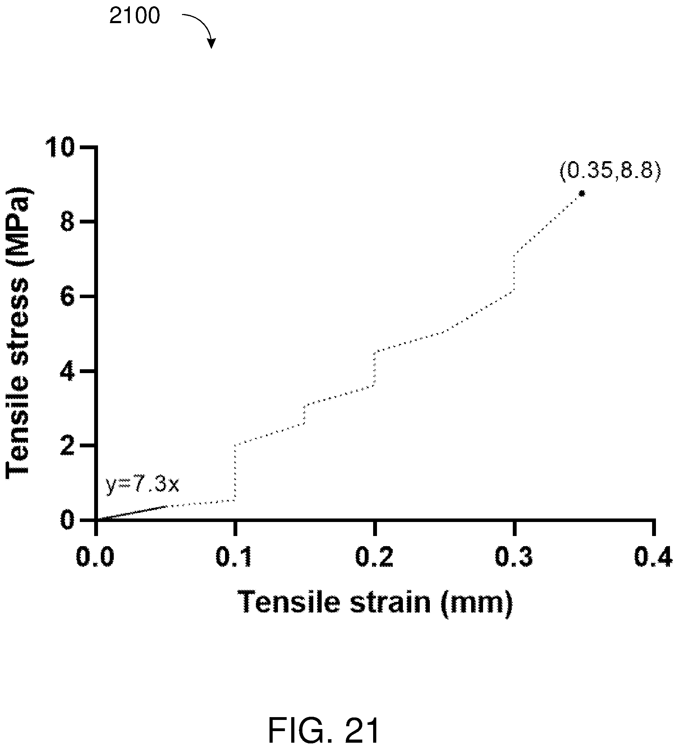

FIG. 21 illustrates a graphical representation 2100 of stress-strain graphs of a PCL-collagen scaffold sample (mass ratio of 49:51) electrospun with the drum collector and crosslinked by 0.2% EDC/NHS solution, obtained with in-house test methods. Young's Modulus is approximately 7.30 MPa and Ultimate Tensile strength is approximately 8.80 MPa (the largest value obtained from all data).

### Second and Third Tests:

### Materials and Methods of Instron testing of tensile strength

Scaffolds were cut into 40 x 10 mm strips. They are then immersed in the crosslinking solution (EDC/NHS) for 5 hours (the first crosslinking). After which, they are washed in normal saline solution (NSS) for 18 hours. The scaffolds are then sent for oxygen plasma treatment. They may be crosslinked a second time for 5 hours after treatment with oxygen plasma (the second crosslinking). The scaffold strips were then loaded on the Instron 3340 machine (Agency for Science, Technology and Research, Singapore), with a 50 N load cell at the crosshead speed of 1 mm/min, in accordance with ASTM D882-02. Young's modulus was then determined from the slope of the Load-Extension curve obtained, and the Ultimate Tensile strength was determined at the highest tensile strength value before the break point.

### Results of Instron testing of tensile strength

**Table 6: Summary table of all tensile testing data conducted via Instron testing. All PCL-COL scaffolds are electrospun at the mass ratio of 49:51 and crosslinked with EDC/NHS for 5 hours. Plasma treatment is set at 30 minutes with the flow rate of Oxygen gas at 50 sccm, frequency at 70 kHz, and the power of 20 W.**

| **Sample** | **Instron Trial** | **Plasma Duration (min)** | **Plasma Oxygen (sccm)** | **Young's Modulus (MPa)** | **Ultimate Tensile Strength (MPa)** |
|---|---|---|---|---|---|
| 1 | 1 | 30 | 50 | 1.13 | 0.54 |
| 2 | 1 | 30 | 50 | 1.07 | 0.18 |
| 3 | 1 | 30 | 50 | 1.52 | 0.33 |
| 4 | 1 | 30 | 50 | 1.47 | 0.23 |
| 5 | 1 | 30 | 50 | 5.93 | 0.57 |
| 6 | 1 | 30 | 50 | 2.57 | 0.82 |
| 7 | 2 | 30 | 50 | 5.27 | 0.43 |
| 8 | 2 | 30 | 50 | 2.31 | 0.72 |
| 9 | 2 | 30 | 50 | 5.85 | 1.56 |
| 10 | 2 | 30 | 50 | 6.96 | 1.45 |
| 11 | 2 | 30 | 50 | 4.20 | 1.81 |
| 12 | 2 | 30 | 50 | 4.00 | 1.99 |
| 13 | 2 | 30 | 50 | 21.82 | 4.93 |
| 14 | 2 | 30 | 50 | 33.23 | 5.21 |
| 15 | 2 | 30 | 50 | 21.98 | 5.66 |
| 16 | 2 | 30 | 50 | 27.93 | 4.14 |
| 17 | 2 | 30 | 50 | 20.12 | 3.66 |
| 18 | 2 | 30 | 50 | 26.40 | 4.12 |

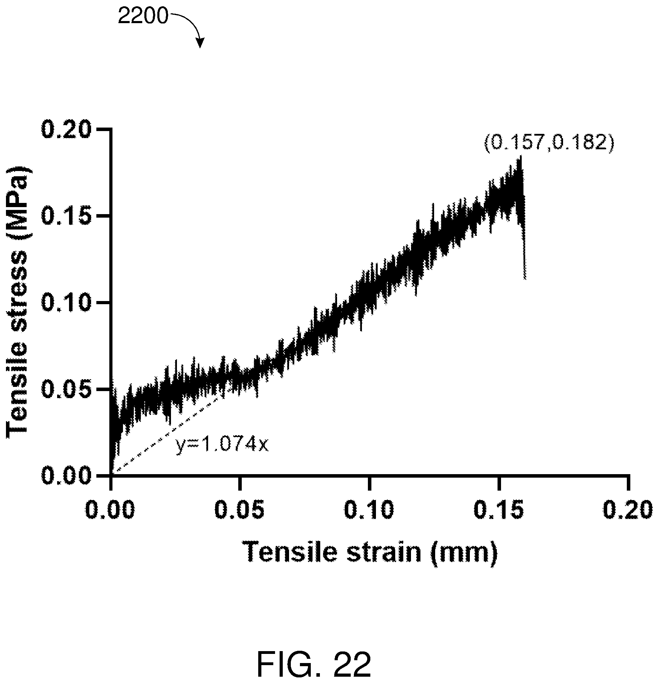

FIG. 22 illustrates a graphical representation 2200 of stress-strain graphs of a PCL-collagen scaffold sample (mass ratio of 49:51) electrospun with the drum collector and the first crosslinked by EDC/NHS solution, obtained from Instron testing trial 1. Young's Modulus is approximately 1.07 MPa (smallest value obtained from all data). Ultimate Tensile strength is approximately 0.18 MPa (smallest value obtained from all data).

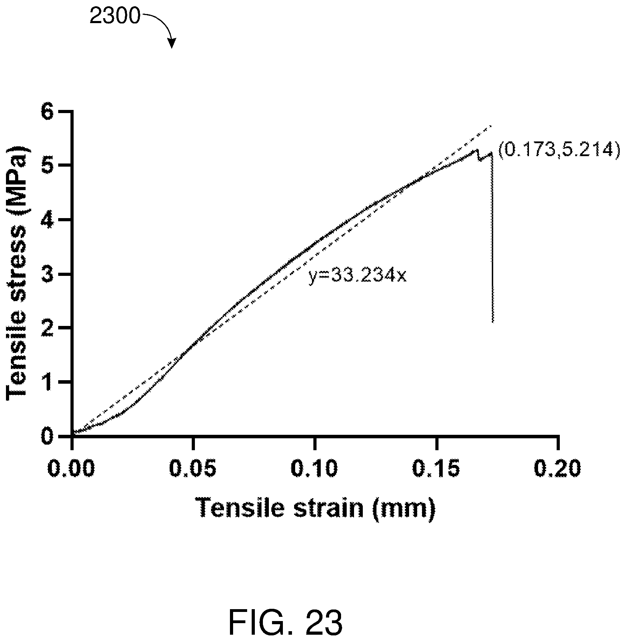

FIG. 23 illustrates a graphical representation 2300 of stress-strain graphs of a PCL-collagen scaffold sample (mass ratio of 49:51) electrospun with the drum collector and the second crosslinked by EDC/NHS solution, obtained from Instron testing trial 2. Young's Modulus is approximately 33.20 MPa (largest value obtained from all data). Ultimate Tensile strength is approximately 5.21 MPa.

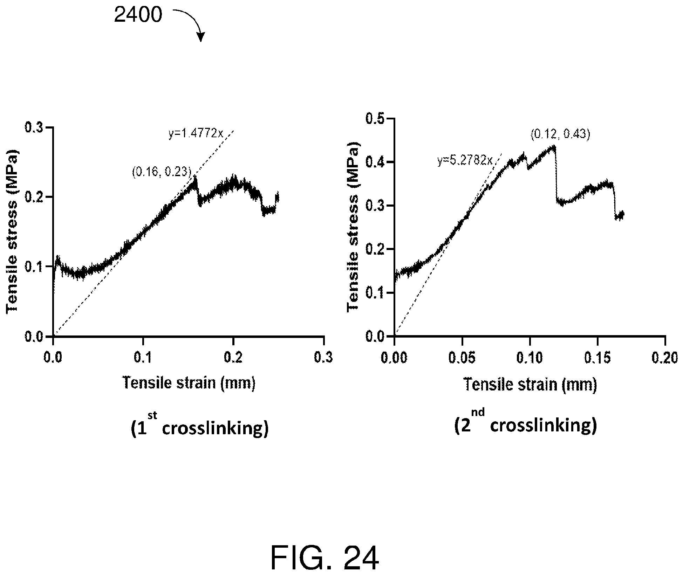

FIG. 24 illustrates a graphical representation 2400 of instron trial 1 average stress-strain graphs of PCL-collagen scaffolds (mass ratio of 49:51) electrospun with the drum collector and crosslinked by EDC/NHS solution. For the first crosslinking (left), Young's Modulus is approximately 1.47 MPa and Ultimate Tensile strength is approximately 0.23 MPa. For the second crosslinking (right), Young's Modulus is approximately 5.27 MPa and Ultimate Tensile strength is approximately 0.43 MPa.

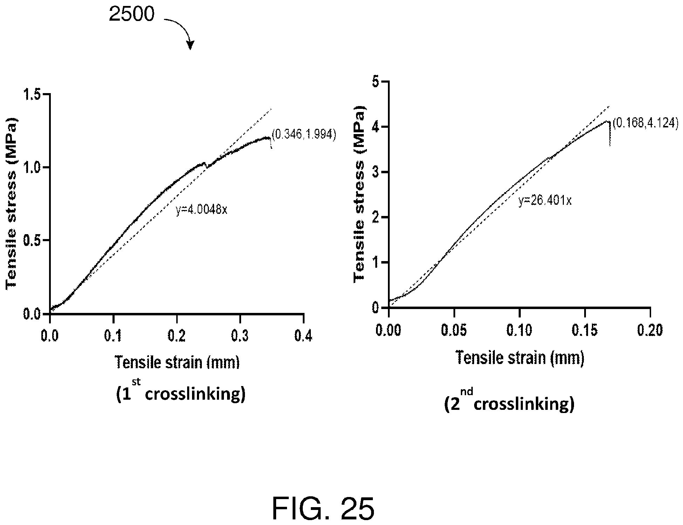

FIG. 25 illustrates a graphical representation 2500 of instron trial 2 average Stress-strain graphs of PCL-collagen scaffolds (mass ratio of 49:51) electrospun with the drum collector and crosslinked by EDC/NHS solution. For the first crosslinking (left), Young's Modulus is approximately 4.00 MPa and Ultimate Tensile strength is approximately 1.99 MPa. For the second crosslinking (right), Young's Modulus is approximately 26.40 MPa and Ultimate Tensile strength is approximately 4.12 MPa.

The value of Young's Modulus (elasticity modulus) of the native cornea is in the range of 0.3 MPa to 7.0 MPa and the value of ultimate tensile strength is less than or equal to 19.1 MPa, obtained by the method of tensile testing. From the data of three separated tensile tests of the scaffold (each layer) according to the present invention, the range of Young's Modulus varied from 1.07 MPa to 33.20 MPa. Even though some data exceeds 7.0 MPa, others are in the range of Young's Modulus of the native cornea (0.3 MPa to 7.0 MPa). The data from these tensile tests also shows that the range of Ultimate Tensile Strength of each layer (from 0.18 MPa to 8.8 MPa) is in the range of ultimate tensile strength of native cornea (≤19.1 MPa). The layer/sheet's capability of remaining intact after handling and suturing confirms that the scaffold according to the present invention is strong enough to withstand load and does not break easily because of the effects of post-treatments (the first crosslinking, plasma treatment, and the second crosslinking) of each layer.

### Animal study

The main objective of this study was to thoroughly assess the safety of the scaffold according to the present invention when used in vivo.

### Methodology of Implantation

### a. Pre-implantation:

Animal's eye examinations were thoroughly performed by the abnormal eye conditions including corneal disorder, elevated intra ocular pressure, ocular adnexa abnormalities will be excluded from the study group if they were found. The implantation area (rabbit eyes) was prepared by given topical gentamicin 4 times daily for 5 days prior to implantation.

### b. Implantation:

After general anesthesia, all surrounding area of the eyeballs including eyelids, skin adjacent to forehead were shaved and cleaned as a routine surgical preparation. Ocular adnexa were cleaned twice with diluted betadine. Ocular surface (cornea and conjunctiva) was rinsed twice with betadine dilution. Rabbit was place in the position of lateral recumbency for performed the surgery. Fixation forceps were used for corneal primary position and lamellar keratoplasty was further performed.

Lamellar keratoplasty is an operation which performs to remove the anterior part of corneal stromal thickness. Trephine of 6.5 mm in diameter (Coronet, UK) was used for punch biopsy at the paracenter of the cornea. Corneal scissors were additionally used to completely generate the recipient bed; corneal pocket of 0.25 mm-thickness. The scaffold according to the present invention was well placed into the corneal pockets and adjusted to cover all the area of created corneal wound (corneal pocket), then sutured with the 9-0 absorbable suture materials with simple interrupted suture pattern. Lamellar keratoplasty was operated on both sides of rabbit eyes. One side was created only the cornea wound which represented as the control side, then another side was the treatment side which the scaffold according to the present invention was placed to replace the anterior part of corneal stromal thickness after lamellar keratoplasty processes.

### c. Post-implantation:

Sub-conjunctival injection of gentamicin and dexamethasone was administered immediately after implantation. Topical gentamicin and artificial tears will be given 4 times daily for a consecutive 2 weeks, together with topical atropine sulfate twice daily. After fluorescein staining test of the cornea has been confirmed negative at day 5 after implantation, topical dexamethasone and cyclosporine will be administered twice daily for a consecutive 3 weeks.

### Blood analysis:

The rabbits were performed blood withdrawal for 3-time point, at pre-implantation (before lamellar keratoplasty procedure, day 0), post-implantation for 28 days, and post-implantation for 56 days. On the 1^{st}- and 2^{nd}-time point, the blood was collected from marginal ear vein of restrained rabbits using 23gauge(G) needle. The blood was divided into EDTA blood collection tube for complete blood count (CBC) analysis and heparin blood collecting tube for blood chemistry profile analysis.

### Results:

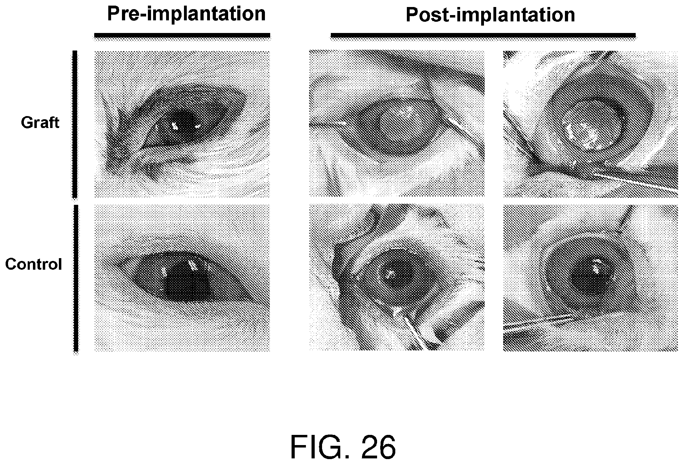

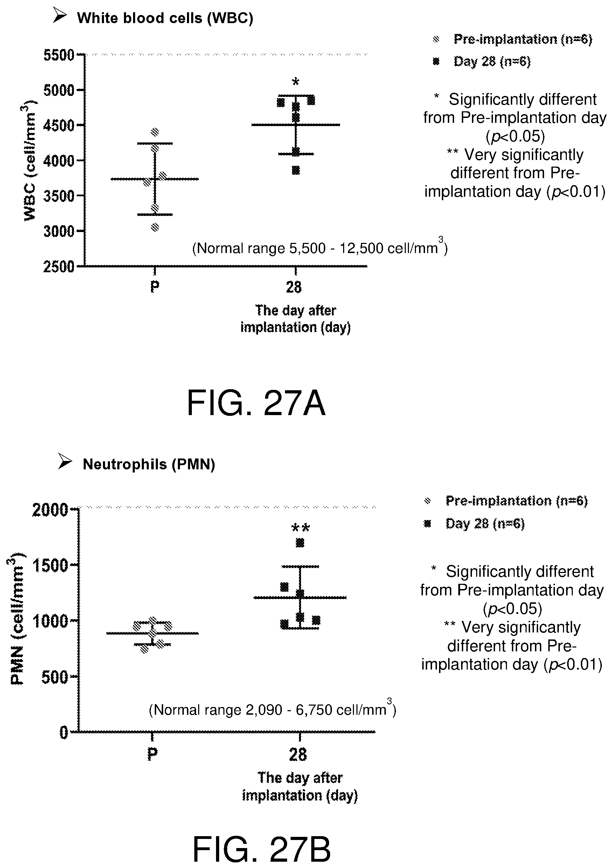

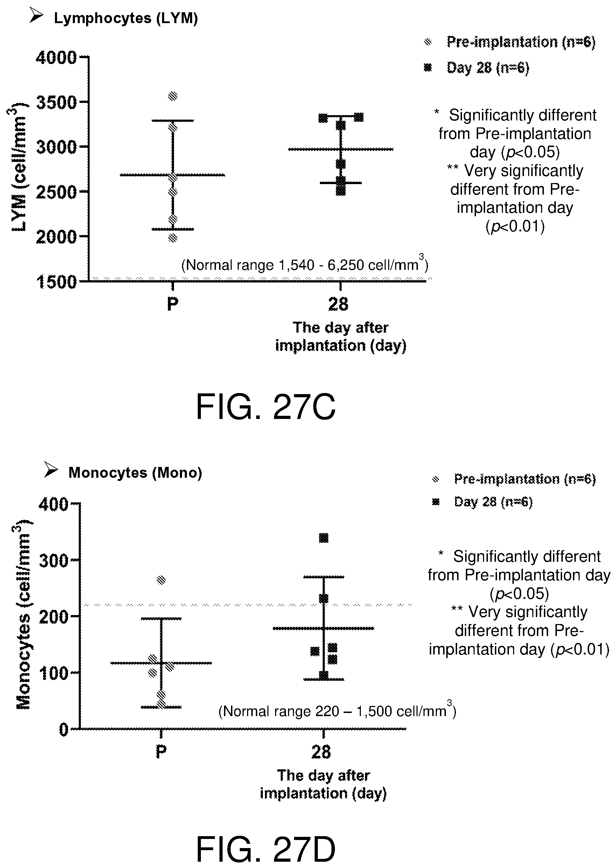

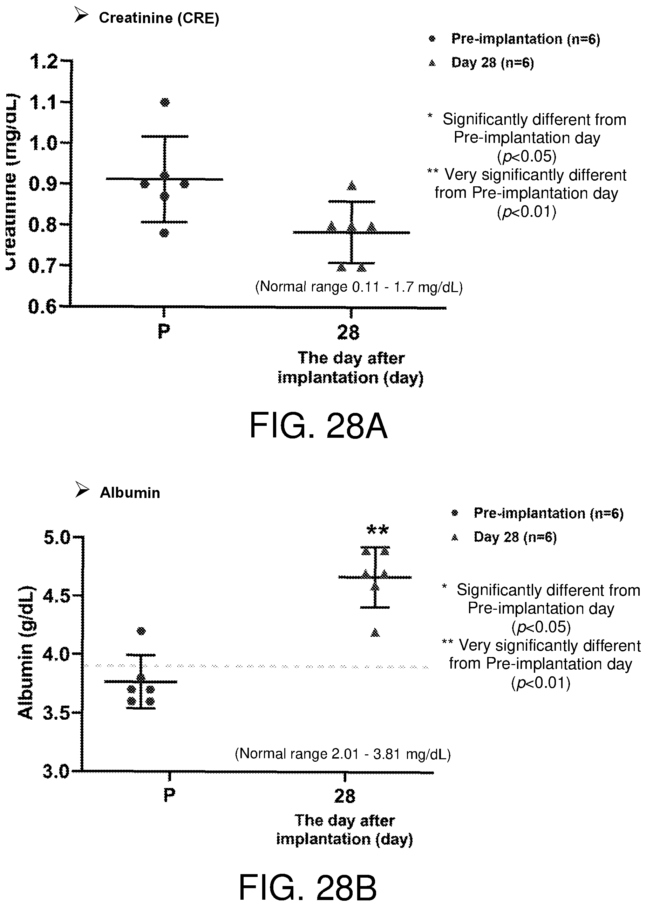

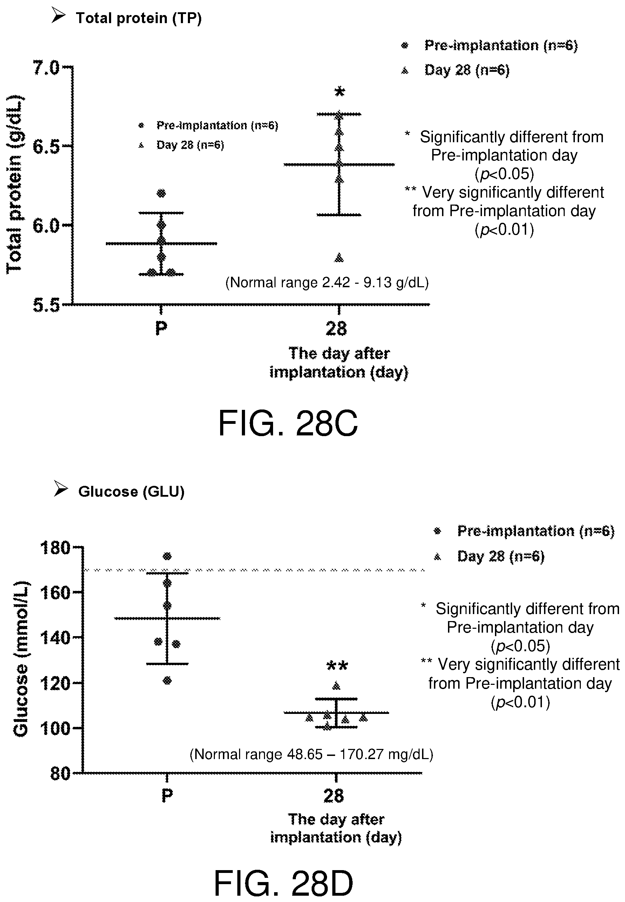

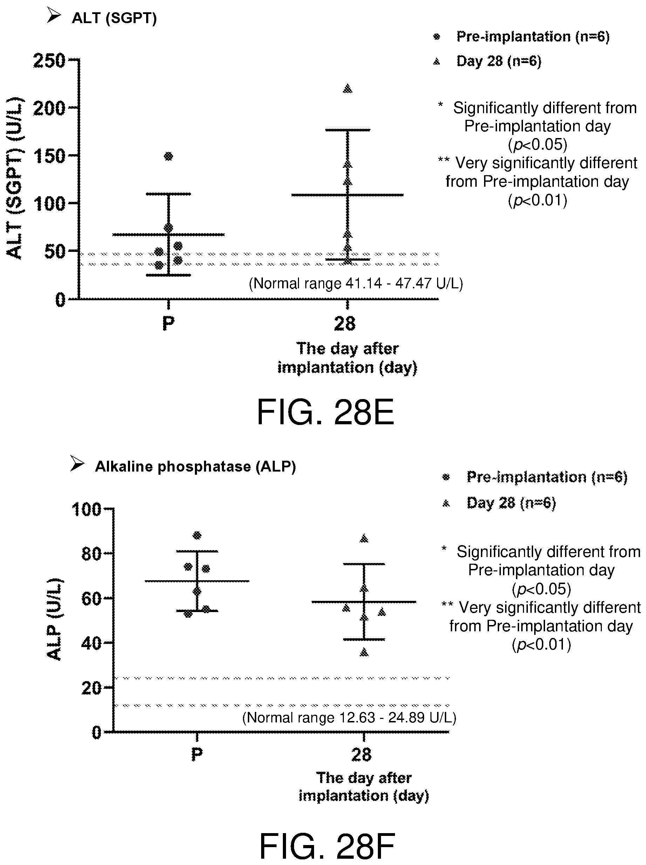

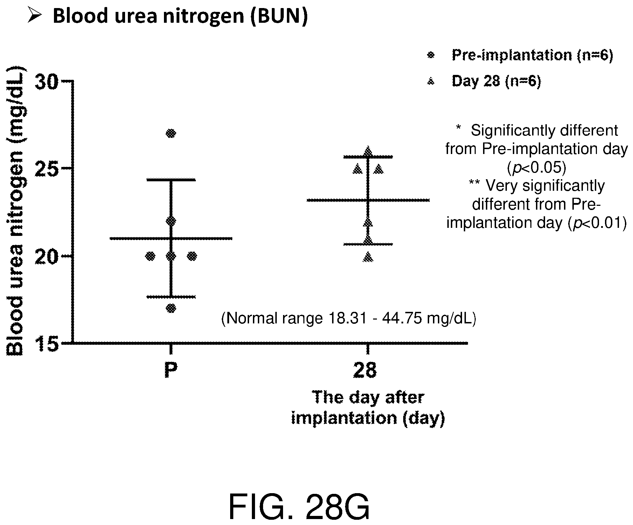

The surgical implantation procedures carried out in rabbit eyes were successful, as depicted in FIG 26. After 28 days of implantation, analysis of complete blood count levels and comprehensive blood chemistry levels in living rabbits were performed. As shown in FIGs. 27A-D and FIGs. 28A-G, the results of the complete blood count levels and comprehensive blood chemistry level indicated that the composition of the scaffold according to the present invention does not induce systemic inflammation. Hence, the results confirm the biomaterial's safety of the scaffold according to the present invention in living mammalian animals.

All terms used in the claims are intended to be given their ordinary meanings as understood by those knowledgeable in the technologies described herein unless an explicit indication to the contrary is made herein. In particular, the use of the singular article such as "a," "the," "said," etc. should be read to recite one or more of the indicated elements unless a claim recites an explicit limitation to the contrary. Conditional language, such as, among others, "can," "could," "might," or "may," unless specifically stated otherwise, or otherwise understood within the context as used, is generally intended to convey that certain embodiments could include, while other embodiments may not include, certain features, elements, and/or steps. Thus, such conditional language is not generally intended to imply that features, elements, and/or steps are in any way required for one or more embodiments.

## Claims

1. A method for producing a scaffold for corneal regeneration, the method comprising a step (a) of deriving a layer of aligned fibers from a solution comprising a biocompatible polycaprolactone (PCL) and a collagen, wherein the PCL: collagen mass ratio is in the range from 77:23 to 42:58, and wherein said method further comprises:
- a step (b) of using a first crosslinker to crosslink the aligned fibers in the layer obtained from the step (a);
- a step (c) of applying a plasma treatment to the aligned fibers in the layer obtained from the step (b); and
- a step (d) of using a second crosslinker to crosslink the aligned fibers in the layer obtained from the step (c).

2. The method according to claim 1, wherein the layer of the aligned fibers has a thickness that is from 15 to 20 µm.

3. The method according to claim 1, further comprises a step (e) of stacking a plurality of the layers of the aligned fibers obtained from the step (d) to produce the scaffold comprising a plurality of the layers of the aligned fibers.

4. The method according to claim 3, wherein the scaffold comprises four layers of the aligned fibers.

5. The method according to claim 1, wherein the first crosslinker is selected from the group consisting of 1-Ethyl-3-(3-dimethylaminopropyl) carbodiimide and N-Hydroxysuccinimide (EDC/NHS), Glutaraldehyde, Riboflavin, Genipin, Paraformaldehyde, and any combination thereof.

6. The method according to claim 5, wherein the first crosslinker is 1-Ethyl-3-(3-dimethylaminopropyl) carbodiimide and N-Hydroxysuccinimide (EDC/NHS).

7. The method according to claim 1, wherein the second crosslinker is selected from the group consisting of 1-Ethyl-3-(3-dimethylaminopropyl) carbodiimide and N-Hydroxysuccinimide (EDC/NHS), Glutaraldehyde, Riboflavin, Genipin, Paraformaldehyde, and any combination thereof.

8. The method according to claim 7, wherein the second crosslinker is 1-Ethyl-3-(3-dimethylaminopropyl) carbodiimide and N-Hydroxysuccinimide (EDC/NHS).

9. The method according to claim 1, wherein the plasma treatment is carried out with an oxygen gas stream at a flow rate of 50 standard cubic centimeters per minute (sccm) and for 20 to 119 minutes.

10. The method according to any one of claims 1 to 9, wherein the scaffold has at least one of the following properties:
(a) a visible light transmittance in the range of 50 to 95%;
(b) a tensile strength less than or equal to 19.1 MPa;
(c) an elasticity modulus in the range of 1 MPa to 34 MPa.

11. A scaffold for corneal regeneration comprising at least one layer of aligned fibers, wherein said at least one layer of aligned fibers is obtainable by a method according to any one of claims 1 to 10.

12. The scaffold according to claim 11, wherein each layer of the aligned fibers has a thickness that is preferably from 15 to 20 µm.

13. The scaffold according to claim 11, wherein said scaffold comprises four layers of the aligned fibers.

14. The scaffold according to claim 11, wherein the collagen is a type I collagen derived from a human collagen, a bovine collagen, a porcine collagen, or an aquatic animal collagen.

15. The scaffold according to claim 14, wherein the type I collagen is preferably an Atelocollagen derived from the bovine collagen.

16. The scaffold according to claim 15, wherein said scaffold has at least one of the following properties:
(a) a visible light transmittance in the range of 50 to 95%;
(b) a tensile strength less than or equal to 19.1 MPa;
(c) an elasticity modulus in the range of 1 MPa to 34 MPa.

## Patentansprüche

1. Verfahren zur Herstellung eines Gerüsts für die Hornhautregeneration, wobei das Verfahren einen Schritt (a) des Ableitens einer Schicht aus ausgerichteten Fasern aus einer Lösung umfasst, die ein biokompatibles Polycaprolacton (PCL) und Kollagen enthält, wobei das Massenverhältnis von PCL zu Kollagen im Bereich von 77:23 bis 42:58 liegt, und wobei das Verfahren ferner umfasst:
- einen Schritt (b) der Verwendung eines ersten Vernetzers, um die ausgerichteten Fasern in der aus Schritt (a) erhaltenen Schicht zu vernetzen;
- einen Schritt (c) des Anwendens einer Plasmabehandlung auf die ausgerichteten Fasern in der aus Schritt (b) erhaltenen Schicht; und
- einen Schritt (d) der Verwendung eines zweiten Vernetzers, um die ausgerichteten Fasern in der aus Schritt (c) erhaltenen Schicht zu vernetzen.

2. Verfahren nach Anspruch 1, wobei die Schicht aus den ausgerichteten Fasern eine Dicke von 15 bis 20 µm aufweist.

3. Verfahren nach Anspruch 1, das ferner einen Schritt (e) des Stapelns einer Vielzahl der aus Schritt (d) erhaltenen Schichten aus den ausgerichteten Fasern umfasst, um das Gerüst herzustellen, das eine Vielzahl der Schichten aus den ausgerichteten Fasern umfasst.

4. Verfahren nach Anspruch 3, wobei das Gerüst vier Schichten der ausgerichteten Fasern umfasst.

5. Verfahren nach Anspruch 1, wobei der erste Vernetzer aus der Gruppe ausgewählt ist, die aus 1-Ethyl-3-(3-dimethylaminopropyl)carbodiimid und N-Hydroxysuccinimid (EDC/NHS), Glutaraldehyd, Riboflavin, Genipin, Paraformaldehyd und beliebigen Kombinationen davon besteht.

6. Verfahren nach Anspruch 5, wobei der erste Vernetzer 1-Ethyl-3-(3-dimethylaminopropyl)carbodiimid und N-Hydroxysuccinimid (EDC/NHS) ist.

7. Verfahren nach Anspruch 1, wobei der zweite Vernetzer aus der Gruppe ausgewählt ist, die aus 1-Ethyl-3-(3-dimethylaminopropyl)carbodiimid und N-Hydroxysuccinimid (EDC/NHS), Glutaraldehyd, Riboflavin, Genipin, Paraformaldehyd und beliebigen Kombinationen davon besteht.

8. Verfahren nach Anspruch 7, wobei der zweite Vernetzer 1-Ethyl-3-(3-dimethylaminopropyl)carbodiimid und N-Hydroxysuccinimid (EDC/NHS) ist.

9. Verfahren nach Anspruch 1, wobei die Plasmabehandlung mit einem Sauerstoffgasstrom bei einer Durchflussrate von 50 Standardkubikzentimetern pro Minute (sccm) und für 20 bis 119 Minuten durchgeführt wird.

10. Verfahren nach einem der Ansprüche 1 bis 9, wobei das Gerüst mindestens eine der folgenden Eigenschaften aufweist:
(a) eine Durchlässigkeit für sichtbares Licht im Bereich von 50 bis 95 %;
(b) eine Zugfestigkeit von kleiner oder gleich 19,1 MPa;
(c) einen Elastizitätsmodul im Bereich von 1 MPa bis 34 MPa.

11. Gerüst zur Hornhautregeneration, das mindestens eine Schicht aus ausgerichteten Fasern umfasst, wobei die mindestens eine Schicht aus ausgerichteten Fasern durch ein Verfahren gemäß einem der Ansprüche 1 bis 10 erhältlich ist.

12. Gerüst gemäß Anspruch 11, wobei jede Schicht der ausgerichteten Fasern eine Dicke hat, die vorzugsweise zwischen 15 und 20 µm liegt.

13. Gerüst gemäß Anspruch 11, wobei das Gerüst vier Schichten der ausgerichteten Fasern umfasst.

14. Gerüst gemäß Anspruch 11, wobei das Kollagen ein Typ-I-Kollagen ist, das von einem menschlichen Kollagen, einem Rinderkollagen, einem Schweinekollagen oder einem Kollagen aus Wassertieren abgeleitet ist.

15. Gerüst gemäß Anspruch 14, wobei das Typ-I-Kollagen vorzugsweise ein Atelokollagen ist, das aus Rinderkollagen abgeleitet ist.

16. Gerüst gemäß Anspruch 15, wobei das Gerüst mindestens eine der folgenden Eigenschaften aufweist:
(a) eine Durchlässigkeit für sichtbares Licht im Bereich von 50 bis 95 %;
(b) eine Zugfestigkeit von kleiner oder gleich 19,1 MPa;
(c) einen Elastizitätsmodul im Bereich von 1 MPa bis 34 MPa.

## Revendications

1. Procédé pour produire un échafaudage destiné à une régénération cornéenne, le procédé comprenant une étape (a) de dérivation d'une couche de fibres alignées à partir d'une solution comprenant une polycaprolactone (PCL) biocompatible et un collagène, dans lequel le rapport en masse PCL/collagène est situé dans la plage allant de 77/23 à 42/58, et dans lequel ledit procédé comprend en outre :
- une étape (b) d'utilisation d'un premier agent de réticulation pour réticuler les fibres alignées dans la couche obtenue à partir de l'étape (a) ;
- une étape (c) d'application d'un traitement par plasma aux fibres alignées dans la couche obtenue à partir de l'étape (b) ; et
- une étape (d) d'utilisation d'un deuxième agent de réticulation pour réticuler les fibres alignées dans la couche obtenue à partir de l'étape (c).

2. Procédé selon la revendication 1, dans lequel la couche de fibres alignées a une épaisseur de 15 à 20 µm.

3. Procédé selon la revendication 1, comprenant en outre une étape (e) d'empilement d'une pluralité des couches de fibres alignées obtenues à partir de l'étape (d) pour produire l'échafaudage comprenant une pluralité des couches de fibres alignées.

4. Procédé selon la revendication 3, dans lequel l'échafaudage comprend quatre couches de fibres alignées.

5. Procédé selon la revendication 1, dans lequel le premier agent de réticulation est choisi dans le groupe constitué par le 1-éthyl-3-(3-diméthylaminopropyl)carbodiimide et N-hydroxysuccinimide (EDC/NHS), le glutaraldéhyde, la riboflavine, la génipine, le paraformaldéhyde, et l'une quelconque de leurs combinaisons.

6. Procédé selon la revendication 5, dans lequel le premier agent de réticulation est le 1-éthyl-3-(3-diméthylaminopropyl)carbodiimide et N-hydroxysuccinimide (EDC/NHS).

7. Procédé selon la revendication 1, dans lequel le deuxième agent de réticulation est choisi dans le groupe constitué par le 1-éthyl-3-(3-diméthylaminopropyl)carbodiimide et N-hydroxysuccinimide (EDC/NHS), le glutaraldéhyde, la riboflavine, la génipine, le paraformaldéhyde, et l'une quelconque de leurs combinaisons.

8. Procédé selon la revendication 7, dans lequel le deuxième agent de réticulation est le 1-éthyl-3-(3-diméthylaminopropyl)carbodiimide et N-hydroxysuccinimide (EDC/NHS).

9. Procédé selon la revendication 1, dans lequel le traitement par plasma est effectué avec un courant d'oxygène gazeux à un débit de 50 normo-centimètres cubes par minute (Ncm³/min) et pendant 20 à 119 minutes.

10. Procédé selon l'une quelconque des revendications 1 à 9, dans lequel l'échafaudage a au moins une des propriétés suivantes :
(a) un coefficient de transmission de la lumière visible situé dans la plage allant de 50 à 95 % ;
(b) une résistance à la traction inférieure ou égale à 19,1 MPa ;
(c) un module d'élasticité situé dans la plage allant de 1 MPa à 34 MPa.

11. Echafaudage destiné à une régénération cornéenne comprenant au moins une couche de fibres alignées, dans lequel ladite au moins une couche de fibres alignées peut être obtenue par un procédé selon l'une quelconque des revendications 1 à 10.

12. Echafaudage selon la revendication 11, dans lequel chaque couche de fibres alignées a une épaisseur qui est de préférence de 15 à 20 µm.

13. Echafaudage selon la revendication 11, dans lequel ledit échafaudage comprend quatre couches de fibres alignées.

14. Echafaudage selon la revendication 11, dans lequel le collagène est un collagène de type I dérivé d'un collagène humain, d'un collagène bovin, d'un collagène porcin, ou d'un collagène d'animal aquatique.

15. Echafaudage selon la revendication 14, dans lequel le collagène de type I est de préférence un atélocollagène dérivé de collagène bovin.

16. Echafaudage selon la revendication 15, dans lequel ledit échafaudage a au moins une des propriétés suivantes :
(a) un coefficient de transmission de la lumière visible situé dans la plage allant de 50 à 95 % ;
(b) une résistance à la traction inférieure ou égale à 19,1 MPa ;
(c) un module d'élasticité situé dans la plage allant de 1 MPa à 34 MPa.
